# EUROPEAN PATENT SPECIFICATION

(11) **EP 1 143 988 B1**
(45) Date of publication and mention of the grant of the patent: **18.02.2004**
(21) Application number: 99957390.0
(22) Date of filing: 25.11.1999
(51) Int. Cl.: A61K 35/78, A23L 1/305, A23L 1/308, A61P 3/10, A61P 3/06, A61P 9/00, A61P 9/10

(54) **COMPOSITION COMPRISING SOY PROTEIN, DIETARY FIBRES AND A PHYTOESTROGEN COMPOUND AND USE THEREOF IN THE PREVENTION AND/OR TREATMENT OF TYPE 2 DIABETES, THE METABOLIC SYNDROME AND ASSOCIATED CARDIOVASCULAR DISEASES**
ZUSAMMENSETZUNG ENTHALTEND SOJAPROTEIN, DIÄTFASERN UND PHYTOESTROGEN SOWIE IHRE VERWENDUNG ZUR VORBEUGUNG UND/ODER BEHANDLUNG VON TYP II DIABETES, DES METABOLISCHEN SYNDROMS UND ASSOZIERTEN KARDIOVASKULÄREN ERKRANKUNGEN
COMPOSITION CONTENANT DES PROTEINES DE SOJA, DES FIBRES ALIMENTAIRES ET UN COMPOSE DE PHYTOESTROGENE ET SON UTILISATION POUR LA PREVENTION ET/OU LE TRAITEMENT DU DIABETE DE TYPE 2, DU SYNDROME METABOLIQUE ET DE MALADIES CARDIOVASCULAIRES APPARENTEES

(30) Priority: 25.11.1998 DK 155698; 16.06.1999 DK 85699
(43) Date of publication of application: 17.10.2001
(73) Proprietor: Nutri Pharma ASA, 0116 Oslo (NO)
(72) Inventor: HOIE, Lars Henrik, London NW8 0LU (GB)
(74) Representative: Kjerrumgaard, Bent
(86) International application number: PCT/IB1999/001992
(87) International publication number: WO 2000/030663

(56) References cited:
- EP-A- 0 898 900
- WO-A-97/37547
- WO-A-98/03084
- FR-A- 2 395 288
- US-A- 5 698 256
- GOVINDJI A.: "Soy: is this a food we could be encouraging in diabetes?" PRACTICAL DIABETES INTERNATIONAL, vol. 15, no. 6, September 1998 (1998-09), pages 163-164, XP000891652

## Description

### FIELD OF THE INVENTION

The present invention relates to use of soy protein, phytoestrogens and dietary fibres and compositions thereof suitable for preventing and/or treating type 2 diabetes and/or the metabolic syndrome. The present invention also pertains to the use of such compositions in the prevention and/or treatment of a cardiovascular disease in a subject suffering from type 2 diabetes. The compositions are particularly useful in preventing and/or reducing the influx of triglycerides and/or cholesterol into the arterial wall of diabetic subjects. The compositions are also useful in lowering serum glucose levels and/or serum levels of cholesterol and/or triglycerides in diabetic subjects. The present invention also relates to the use of these compositions as a medicament and/or in the manufacture of a medicament for treating type 2 diabetes and/or the metabolic syndrome and/or a cardiovascular disease in a subject suffering from type 2 diabetes.

### BACKGROUND OF THE INVENTION

Serum glucose levels vary quite significantly depending on the nutritional status of a subject. Following a dietary intake rich in glucose containing carbohydrates, several homeostatic mechanisms are capable of promoting glucose uptake into cells as well as facilitating the metabolism of glucose leading e.g. to the synthesis of glycogen in the liver and muscles. When glucose levels subsequently fall some time after a meal, other regulatory mechanisms initiates the release of glucose from glycogen and initiates gluconeogenesis in order to maintain the blood glucose levels within the required limits.

Some homeostatic mechanisms are dependent on the action of hormones, and the most important hormone promoting glucose uptake and metabolism is insulin. In contrast, other hormones such as e.g. glucagon and epinephrine act antagonistically and facilitate increased blood glucose levels. Insulin is synthesized in pancreatic β cells and secreted in response to e.g. increased levels of blood glucose.

Once secreted into the bloodstream, insulin acts in several processes to promote i) uptake of metabolizable substrates into certain cells, ii) storage of lipids and glycogen, and iii) biosynthesis of macromolecules such as nucleic acids and proteins. More specifically, the action of insulin results in i) an increased uptake of glucose in muscles and adipose tissues, ii) activation of the glycolytic pathway in the liver, iii) an increased synthesis of fatty acids and triglycerides in the liver and adipose tissues, iv) inhibition of lipolysis, v) inhibition of gluconeogenesis in liver, vi) an increased glycogen synthesis in liver and muscle tissue, vii) stimulation of amino acid uptake, viii) an increased protein synthesis in muscles, and ix) inhibition of proteolysis.

The blood glucose elevation occurring shortly after an intake of a meal rich in carbohydrates stimulates the secretion of insulin and concomitantly suppresses the secretion of glucagon. The combined effect thereof is a promotion of uptake of glucose into the liver, stimulation of glycogen synthesis and suppression of glycogen breakdown. When blood glucose levels subsequently begin to fall, the above events are reversed. A decreased secretion of insulin and an increased secretion of glucagon lead to the breakdown of glycogen in the liver and triglycerides in adipocytes. Triglycerides are converted into fatty acids that are used by hepatic and muscle tissues. At the same time, the decreased insulin levels serve to reduce glucose utilization by hepatic, muscle and adipose tissues.

In type 2 diabetes, an impaired insulin secretion as well as decreased insulin sensitivity is present. As a result of this, glucose is present in excessive amounts in the bloodstream in association with either low, normal or even high insulin levels.

Type 2 diabetes accounts for approximately 80-90% of all diabetes cases and is arguably the fastest growing global threat to public health. Left unchecked, the current trend has been estimated to result in 215 million sufferers from type 2 diabetes worldwide by the year 2010.

Various clinical studies have implicated obesity as a risk factor for type 2 diabetes although the underlying mechanism for its role in the pathogenesis of the disease is still unclear. Obesity amongst people, who subsequently develop type 2 diabetes, as well as those with existing type 2 diabetes, is associated with an increased hepatic output and reduced glucose utilization by peripheral tissues, such as e.g. muscles.

Fatty acid metabolism is increased in both obesity and in type 2 diabetes, and this may affect glucose utilization by interfering with the actions of insulin.

The development of type 2 diabetes is progressive and likely to be a culmination of pathophysiological changes occurring over many years. In most cases, the subject is unaware of the disease process, particularly in the early stages. The first stage of the disease is thought to be initiated due to a resistance to insulin. Insulin resistance is strongly associated with, and probably a major contributor to, the disease eventually entering the diabetic state. The insulin resistance stage is characterised by reduced sensitivity to insulin, as the cells normally stimulated by insulin are less sensitive to the hormone. The next stage of the disease is that of impaired glucose tolerance (IGT). IGT follows from a continued increase in insulin resistance, i.e. a continued decrease in insulin sensitivity. Impaired glucose tolerance is formally defined as a fasting venous plasma glucose concentration < 7.0 mmol/1 (126 mg/dl) and a two-hour venous plasma value after 75 gram oral glucose intake ≥ 7.8 (≥ 140) and < 11,1 mmol/l (< 200 mg/dl). When glucose concentration ≥ 11.1 mmol/1, i.e. a level indicative of type 2 diabetes, the risk of developing specific diabetic complications is greatly enhanced. However, IGT and type 2 diabetes are both associated with a 2-4 fold increase in the burden of cardiovascular diseases. The World Health Organization (WHO) glycaemic criteria have been applied in a number of studies of IGT. These studies have determined the rate of progression to type 2 diabetes - 5 to 10 years after detection of IGT - at between 19% and 61%.

The final phase of type 2 diabetes development is characterised by insulin secretory failure (ISF). In this stage of type 2 diabetes, the insulin secretory response is inadequate. It is believed that this results from impairment of the pancreatic β-cell functions and/or the inability of β-cells to secrete sufficient amounts of insulin to compensate for the increased insulin resistance. It is to be understood that all of the above mentioned phases in the development of type 2 diabetes leading to overt diabetes will be considered as being comprised by the term type 2 diabetes as used herein. Accordingly, a diagnosis of impaired glucose tolerance and/or reduced insulin sensitivity will be understood to relate to an individual also diagnosed as suffering from type 2 diabetes, or a condition, including any precondition, leading to type 2 diabetes.

The progression from normal glucose tolerance (NGT) to impaired glucose tolerance (IGT) is characterised by i) an increasing insulin resistance or a decreasing insulin sensitivity and ii) gradual increases in both fasting and glucose-stimulated plasma insulin levels. As IGT gradually progresses to a mild fasting hyperglycaemia, there may be a further small increase in insulin resistance in both fasting and glucose-stimulated insulin release. However, in this situation, further increases in the rate of insulin secretion are no longer sustained and overt fasting hyperglycaemia and increased post-load glucose intolerance start to emerge.

Many cardiovascular risk factors are abnormally high in individuals suffering from type 2 diabetes and they are consequently at an increased risk of developing various arteriosclerotic vascular diseases. At the time of diagnosis of type 2 diabetes, the existence of arteriosclerotic manifestations is already pronounced in many individuals and may include hyperlipidaemia, hypercholesterolaemia, hypertriglyceridaemia, hyperglycaemia, hypertension, and hyperinsulinaemia.

Arteriosclerosis is a common term for a group of conditions related to the arterial system and leading to an increased arterial wall thickness and a subsequent loss of elasticity. Three main groups of arteriosclerosis frequently referred to are atherosclerosis, Mönckeberg's mediasclerosis and arteriolosclerosis. Atherosclerosis is most frequently observed in the aorta and in the main arteries connected thereto, in the coronary arteries and in the arteries of the brain. Mönckeberg's mediasclerosis leads to a narrowing of the media of the arteries of the extremities, and arteriolosclerosis is related to a narrowing of the small arteries and arterioles caused mainly by hypertension.

One commonly occurring arterial condition in diabetics is that of atherosclerotic cardiovascular disease. The condition may eventually progress through several stages. A normal structure of an artery is characterized by discrete focal numbers of adhering monocytes, some intimal foam cells, and some intimal smooth muscle cells or intimal cell masses at bifurcations. A fatty streak may occur non-symptomatically and involve a layer of foam cells. As arteriosclerosis progresses, the cells making up the inner wall of an artery will gradually start to harden due to the deposition of plaque and the cells may eventually become degenerated. As the wall of an artery thickens, hardens, and lose its elasticity during arteriosclerosis, the blood vessel channels may develop twists and turns and become narrowed so that the heart must work harder to pump the usual amount of blood through the arteries. The condition may regress or it may evolve into a formation of e.g. fibrous plaque. Fibrous plaque is a slowly reversible condition that may evolve to become a complicated lesion.

The cellular degeneration will most likely result in a fracture of the arterial wall which in turn leads to a deposition of calcium, platelet formation and a gradual development of scar tissue further contributing to both cellular degeneration and a substantially reduced elasticity of the arterial wall. Atherosclerosis characterised by a restricted flow of blood through a coronary artery may lead to the development of coronary heart disease. A complicated lesion of an artery is often symptomatic, hardly reversible and may, in severe cases such as thrombosis, be lethal. If a blood clot forms in a coronary artery, the interruption of the blood flow may result in the death of part of the heart muscle and cause the extremely painful chest pains associated with a heart attack.

Accordingly, even though it is well established that cholesterol, lipids and lipoproteins all contribute to the progression of various arteriosclerotic conditions in individuals suffering from diabetes, little is known about the causes of arteriosclerosis. There is at present no simple cure or medical treatment for arteriosclerosis and no effective preventive therapy for treating diabetics suffering from the symptoms of cardiovascular diseases exists.

The effect of the development of arteriosclerosis in diabetes is very dear. The proportion of total deaths from coronary heart disease (CHD) in diabetes has progressively increased and is now reported to cause almost 75 percent of all deaths among type 2 diabetics. However, even though the association between diabetes, insulin resistance, blood lipids, hypertension and premature arteriosclerosis have long been recognised, the complex mechanisms responsible for this association are still as vague and evasive as they were more than 50 years ago.

For type 2 diabetes there has actually not occurred a medical breakthrough since i) insulin was discovered in 1922 and since ii) sulphonylureas, biguanides, and α-glucosidase inhibitors were developed in 1954, 1957 and 1986, respectively. Obesity and insufficient physical exercise have been suggested to be major contributors to type 2 diabetes, and coronary heart diseases and heart infarction are among the most common cardiovascular conditions diagnosed in diabetic subjects. It has been estimated that two out of every three diabetics contract a cardiovascular disease.

An increased serum level of triglycerides is a prominent risk factor for cardiovascular diseases in a diabetic subject. Importantly, recent studies have indicated that serum levels of triglycerides currently considered as "normal" - (2.2 mmol/l) or 200 mg per deciliter of serum - may in fact be too high. It has been proposed that the "normal" limit for triglycerides should be reduced by as much as 50 percent as compared to the limit presently regarded as being the "normal" limit (Yahoo News, 1 May 1998). In a group of patients examined over almost 20 years, a serum triglyceride level of more than 1.1 mmol/l or 100 mg per deciliter serum actually increased the relative risk of contracting a new cardiovascular event by 50 percent and reduced the chance of surviving that event. It was emphasized that so far no clinical trials have examined whether lowering triglyceride levels affects the incidence of subsequent cardiovascular events, and research into the effect of serum triglyceride levels on cardiovascular events lags far behind research directed to establishing the effect of increased cholesterol serum levels on the subsequent development of cardiovascular diseases.

Adlercreutz (Finnish Medical Society, Ann. Med. 29, 95-120 (1997)) stressed that no definite recommendations can be made as to the dietary amounts of soy isoflavones needed for prevention of disease. It is emphasized that phytoestrogens, particularly in association with soy intake, seem to have a great potential for preventing cardiovascular diseases, but as the area is really in the early stages of development, an established beneficial effect of soy and isoflavonoids in this respect will have to await further studies. It is further stated that despite an abundant literature at this early stage of dietary phytoestrogen research, much work is needed before any recommendation as to phytoestrogen consumption can be made. However, experimental and epidemiological evidence does support the view that these compounds do not have any negative effects and that they may form a group of substances with a great potential in preventive medicine. It is emphasized that at present, no definite recommendations can be made as to the dietary amounts needed for disease prevention. No reference is made to a composition comprising a combination of soy protein, a high content of a phytoestrogen compound, and dietary fibres.

Anderson (N. Eng. J. Med. 333, 276-282(1995)) analysed a total of 38 clinical trials and concluded that the consumption of soy protein significantly decreases serum levels of total cholesterol, LDL-cholesterol and triglycerides. It was found that ingestion of diets containing soy protein, as compared with control diets, was accompanied by a significant reduction in serum concentrations of total cholesterol, LDL-cholesterol and triglycerides. However, soy protein intake did not significantly affect serum HDL-cholesterol concentrations. Various types of soy proteins were studied, such as isolated soy protein, textured soy protein, or a combination thereof, and it was found that the type of soy protein did not have any significant effect on the net change in serum cholesterol levels. The study did not consider a simultaneous intake of the various types of soy proteins along with dietary fibres. No reference is made to a composition comprising a combination of soy protein, a high content of a phytoestrogen compound, and dietary fibres.

Hendrich (J. Nutr. 124(9 Suppl.), 1789S-1792S (1994)) has reported that isoflavones may be of great potential benefit to human health maintenance and that isoflavones may be health-protective in amounts potentially available from a human diet containing daily soy foods. The food content of isoflavones is in the range of from 0.1 to 1 mg/g in soy foods. Several factors such as variety of soybean, processing and the addition of other ingredients to the food, influence isoflavone contents of foods. It is stated that human intestinal bacteria can destroy ingested isoflavones to a great extent and that this may be why only 15 to 20 percent of isoflavones are reported to be recoverable in intact form from the urine and faeces. It is emphasized that much work remains to determine the relation between concentration of isoflavones in human urine and plasma and the biological effects of the isoflavones. It is noted that although more health-related animal data need to be obtained, the time is approaching when long-term human feeding trials of purified isoflavones and foods containing isoflavones to examine health-related outcomes may be warranted. No reference is made to a composition comprising a combination of soy protein, a high content of a phytoestrogen compound, and dietary fibres.

Knight (Maturitas 22, 167-175 (1995)) provides a synopsis of the literature relating principally to the clinical effects of phytoestrogens on the diseases associated with ageing. A review of literature pertaining to cardiovascular diseases states that the protective effects of phytoestrogens are manifested through lipid changes, a decrease in LDL-cholesterol and an increase in HDL-cholesterol, and vascular effects, concerning both vasomotor tone and vessel wall compliance. The consumption of soy protein is reported to alter lipid levels and dietary soy protein appears to be anti-atherogenic when compared with various animal proteins. It is concluded that isoflavones represent a large and exciting group of compounds with potential benefits to many diseases in diabetes. It is emphasized that current evidence justifies the conclusion that phytoestrogens may be among the dietary factors affording protective effects against heart disease. However, further clinical studies are required to more clearly elucidate their effects. No reference is made to a composition comprising a combination of soy protein, a high content of a phytoestrogen compound, and dietary fibres.

Knight (Obstet. Gynecol. 87, 897-904 (1996)) has reviewed the sources, metabolism, potencies, and clinical effects of phytoestrogens on humans. The review suggests that phytoestrogens are among the dietary factors affording protection against heart disease in vegetarians. Based on epidemiological and cell line studies, it is emphasized that intervention studies are now an appropriate consideration to assess the clinical effects of phytoestrogens because of the potentially important health benefits associated with the consumption of foods containing these compounds. It is concluded that clinical applications for phytoestrogens are still in their infancy. No reference is made to a composition comprising a combination of soy protein, a high content of a phytoestrogen compound, and dietary fibres.

Packard (Arterioscler. Thromb. Vasc. Biol. 17, 3542-3556 (1997)) has reviewed the heterogeneity in the apoB containing lipoprotein classes and provides an interpretation of kinetic studies of apoB metabolism in the light of underlying structural and functional variations. The review is based on the fact that lipoprotein classes are composed of a limited number of components with distinct properties. However, the basis for this heterogeneity and the consequences for disease are not thoroughly understood. The LDL-fraction is made up of a small number of subtypes of particles with relatively discrete size and density. Subjects with a preponderance of small-sized LDL have a three-fold increased risk of having a myocardial infarction independent of the total concentration of LDL present. No reference is made to a composition comprising a combination of soy protein, a high content of a phytoestrogen compound, and dietary fibres.

Potter (Am. J. Clin. Nutr. 58, 501-506 (1993)) studied the effects of soy protein consumption with and without soy fibre on plasma lipids in mildly hypercholesterolemic men. It was reported that total and LDL-cholesterol concentrations can be lowered significantly in mildly hypercholesterolemic men, as indicated by a replacement of 50 percent of dietary protein with soy protein. Similar reductions in blood lipids were noted for isolated soy protein, whether it was consumed in conjunction with soy cotyledon fibre or cellulose fibre. Plasma triglyceride concentrations were unaffected by the various dietary treatments described in the article. The study did not reveal any additive cholesterol-lowering effect of concurrent intake of cotyledon soy fibre with isolated soy protein, and it was stated that whether or not there is an added benefit in lowering blood cholesterol concentrations from increased concurrent intake of soy protein and fibre in humans is not known. No reference is made to a composition comprising a combination of soy protein, a high content of a phytoestrogen compound, and dietary fibres.

Reinli (Nutr. Cancer 26, 123-148 (1996)) has reviewed the literature for quantitative data on the levels of known phytoestrogens (daidzein, genistein, coumestrol, formononetin and biochanin A) in food plants. It is reported that the isoflavones daidzein and genistein may exist in four related chemical structures, i.e. an aglycone structure (daidzein and genistein), an 7-O-glucoside structure (daidzin and genistin), an 6'-O-acetylglucoside structure (6'-O-acetyldaidzin and 6'-O-acetylgenistin), and a 6'-O-malonylglucoside structure (6'-O-malonyldaidzin and 6'-O-malonylgenistin). The conjugates (7-O-glucosides, 6'-O-acetylglucosides, and 6'-O-malonylglucosides) are transformed to aglycones, which are sometimes called free isoflavones, through hydrolysis in the intestinal tract by β-glucosidase enzymes of gut bacteria. Acid hydrolysis in the stomach may also contribute to the formation of free isoflavones. It is unclear how readily conjugates undergo intestinal hydrolysis and subsequent absorption. It is stressed that isoflavones are metabolised differently by different animals and humans. No reference is made to a composition comprising a combination of soy protein, a high content of a phytoestrogen compound, and dietary fibres.

Sniderman (Am J. Cardiol. 79, 64-67 (1997)) presents a risk factor hypothesis with an emphasis on the integral role of LDL in atherogenesis. It is stressed that a measurement of LDL-cholesterol is an incomplete estimate of the risk attributable to LDL and that other classic risk factors such as e.g. hypertension, diabetes, and smoking exert their proatherogenic potential largely or exclusively by multiplying the malign influences of LDL on the arterial wall. It is acknowledged that small, dense LDL-particles are one of the most common dyslipoproteinaemias associated with coronary artery disease. It is reported that elevated levels of lipoprotein (a) are associated with increased coronary risk, but the basis for this is still not clear. No reference is made to a composition comprising a combination of soy protein, a high content of a phytoestrogen compound, and dietary fibres.

WO 95/10512 relates to a process for producing an aglucone isoflavone enriched vegetable protein whey and discloses in one embodiment a whey having a dry basis genistein content of about 2.6 to about 8.7 mg/gram and a dry basis daidzein content of about 2.5 to about 6.0 mg/gram. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

WO 95/10529 relates to a process for producing an aglucone isoflavone enriched protein concentrate and discloses in one embodiment a concentrate having on a dry basis a genistein content of about 1.0 to about 2.0 mg/gram and a daidzein content of about 0.7 to about 1.5 mg/gram. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

WO 95/10530 relates to a process for producing an aqueous extract comprising protein and glucone isoflavones and discloses in one embodiment a vegetable protein isolate having a dry basis genistein content of about 1.5 to about 3.5 mg/gram and a dry basis daidzein content of about 1.0 to about 3.0 mg/gram. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

WO 97/31546 discloses data from total replacement programmes (for 6 weeks) in weight reduction studies conducted at Karolinska Hospital in Sweden. It is shown that products comprising isolated soy protein and soybean cotyledon fibres reduce serum cholesterol levels by a maximum of 27 percent and triglyceride levels by a maximum of 44 percent for a patient population with a mean initial cholesterol content of 5.6 mmol/l. A mean value of 6.25 mmol/l was determined for all patients having serum cholesterol levels above 6 mmol/l, and for this group of patients a reduction in serum cholesterol levels of 33 percent was observed. Since the reported data were part of a weight reduction programme, a dietary effect and/or an effect related to a weight loss would have contributed to the observed reductions in cholesterol and/or triglycerides. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

WO 97/37547 discloses an isoflavone-enriched soy protein product having a protein content greater than 60 percent of total dry matter, a total dietary fibre content of less than 4 percent of total dry matter, a sucrose content greater than 10 percent of total dry matter, a total content of sulphur-containing amino acids greater than 2.2 percent of the total amino acid content, a stachyose content of less than 1.5 percent of total dry matter, and a total isoflavone content greater than 2.5 mg/gram, equivalent to 0.25 percent. The use of soy cotyledon fibres is not anticipated and the claimed invention is for use as an ingredient in the production of an edible product and not in a treatment of diabetes. Also, the product differs from the present invention by comprising total dietary fibre in an amount of less than 4 percent of total dry matter, and by containing an unusually low amount of stachyose and a high amount of sulphur-containing amino acids. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

US 5,320,949 discloses a process for producing an aglucone isoflavone enriched fibre product from a vegetable protein material in the form of a slurry and discloses in one embodiment an aglucone enriched fibre product directly obtainable from said process and having a genistein content of about 1.0 and 2.0 mg/gram and a daidzein content of about 0.7 to 1.7 mg/gram. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

US 5,352,384 discloses an aglucone enriched fibre product having a genistein content of about 1.0 to 2.0 mg/gram and a daidzein content of about 0.7 to 1.7 mg/gram. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

EP 827 698 A2 and EP 827 698 A3 disclose a process for producing an aglucone isoflavone enriched extract from a vegetable material containing isoflavone conjugates and protein. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

An abstract presented at the American Heart Association's 38^{th} Annual Conference on Cardiovascular Disease Epidemiology and Prevention held in March 1998 disclosed a reduction in the levels of total and LDL-cholesterol in a subject following intake of a diet supplemented with 25 grams of soy protein containing 4 mg, 25 mg, 42 mg, and 58 mg of isoflavones, respectively. A "dose-response" effect was reported so that increasing amounts of isoflavones were associated with an increasing reduction of cholesterol. A maximum reduction of serum levels of total and LDL-cholesterol of 4 percent and 7 percent, respectively, was reported for the product containing 58 mg of isoflavone. No reference is made to a treatment of diabetes by using a composition comprising a combination of soy protein, a high content of a phytoestrogen compound, and dietary fibres. No reference is made to a composition comprising a combination of soy protein, a high content of a phytoestrogen compound, and dietary fibres.

### SUMMARY OF THE INVENTION

The present invention provides use of a nutritional composition having a fixed, high amount of a phytoestrogen compound such as e.g. naturally occurring isoflavones in the prevention and/or treatment of diabetes and cardiovascular disease in diabetic subjects. More particularly the present invention provides use of a nutritional composition of soybean extractable ingredients having a fixed, high amount of a phytoestrogen compound such as e.g. naturally occurring isoflavones in the prevention and/or treatment of diabetes and cardiovascular disease in diabetic subjects.

The present invention represents a potential new breakthrough in diabetes treatment and more particularly, the present invention provides use of a combination comprising a) soy protein, preferably isolated soy protein, b) a high, fixed content of a-plant hormone in the form of a phytoestrogen compound, preferably naturally occurring isoflavones, and c) dietary fibres, preferably soybean fibres, more preferably soybean fibres manufactured from the cotyledon of soybeans hereinafter referred to as soy cotyledon fibres.

The present invention is useful in the prevention and/or treatment of type 2 diabetes and/or a cardiovascular disease in diabetic subjects. Accordingly, use of a composition according to the present invention is effective in lowering serum levels of both glucose and cholesterol and/or triglycerides. No treatment is currently available for concomitantly lowering serum levels of glucose as well as lipid serum levels of total cholesterol and/or LDL-cholesterol and/or triglycerides. It is to be understood that diabetic subjects according to the present invention have a fasting plasma glucose ≥ 7.0 mmol/l.

Use of a composition according to the present invention represents a new approach to treatment of type 2 diabetes and is believed to be capable of i) lowering serum levels of glucose, ii) lowering serum levels of insulin, iii) lowering total serum cholesterol and/or LDL-cholesterol and/or triglyceride levels, iv) increasing glucose tolerance and/or insulin sensitivity and/or v) preventing and/or alleviating and/or treating impaired glucose tolerance and/or insulin secretory failure in diabetic subjects and/or vi) preventing and/or alleviating and/or treating an arteriosclerotic condition by reducing the influx of cholesterol and/or triglycerides into the endocelium of the arterial wall of a diabetic subject suffering from a cardiovascular disease. No other known. compositions are effective in lowering serum levels of both lipids and glucose and/or reducing the influx of lipids such as e.g. cholesterol and/or triglycerides into the arterial wall. Use of a composition according to the present invention may be in the form of a micronutrient as defined herein below.

Phytoestrogen compounds are naturally occurring plant hormones showing a structural similarity to 17β-estradiol. Phytoestrogens consist of a number of classes including isoflavones, coumestans, lignans and resorcylic acid lactones. The class of isoflavones consists of among others genistein, daidzein, equol, glycitein, biochanin A, formononetin, and O-desmethylangolesin. The isoflavones genistein and daidzein are found almost uniquely in soybeans. When present in the plant the isoflavones are mainly in a glucoside form, i.e. attached to a sugar molecule. Isoflavones in this glucoside form can be deconjugated to yield isoflavones in a so-called aglycone form, which is the biologically more active form of isoflavones and which is absorbed faster and to a greater extent in the human gut than isoflavones in the glucoside form. *In vitro* studies have examined the relative estrogenic effect exerted by various phytoestrogens including isoflavones. The resulting potencies as compared to estradiol (having a relative potency of 100), have been reported by Knight (Maturitas 22, 167-175 (1995)) for among others genistein (0.084) and daidzein (0.013). However, the results also showed that the estrogen receptor complexes formed by estradiol and isoflavones such as genistein and daidzein are functionally equivalent. The comparative dissociation constant of genistein for the estrogen receptor, as determined in competitive binding assays, was found to be from 100 to 10.000 times higher than that of estradiol.

The term "naturally occurring" substance as used in the present specification and the appended claims refers to a substance originally isolated from a natural source, such as an animal or a plant, for example a soy plant, or modified forms of such a substance. The naturally occurring substance for use in a composition according to the present invention may be included in a composition according to the present invention as part of the natural source or in any type of extract, isolate or the like thereof, or it may have been isolated from a plant source or synthesized biologically, microbiologically, or chemically or by any other means.

Soy proteins are involved in a lowering of cholesterol and triglyceride levels, they are easily digestible, and they represent an efficient sole protein source for maintaining the nitrogen balance. Soy isoflavones in high intakes further enhances this effect. Dietary fibres, such as soybean fibres, especially soy cotyledon fibres have been shown to lower total serum cholesterol levels, to improve glucose tolerance, to increase insulin sensitivity, to normalise the gastrointestinal function, and to exert no influence on the absorption of essential minerals.

Accordingly, in one aspect the present invention provides use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 peroent of the total energy content of the composition,
(b) at least one phytoestrogen compound in an amount of more than 0.16 weight percent of the soy protein content of the composition, and
(c) dietary fibres in an amount of more than 6 weight percent of the total weight of the composition on a dry basis,
for the manufacture of a nutritional composition for lowering serum levels of glucose and/or total cholesterol and/or LDL-cholesterol and/or triglycerides in a diabetic subject.

In a more preferred aspect the present invention provides a composition comprising
(a) isolated soy protein in an amount of at least 50 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) soybean fibres in an amount of more than 6 weight percent of the total weight of the composition on a dry basis,
for the manufacture of a nutritional composition for lowering serum levels of glucose and/or total cholesterol and/or LDL-cholesterol and/or triglycerides in a diabetic subject.

In a most preferred aspect the present invention provides a composition comprising
(a) isolated soy protein in an amount of at least 50 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) soy cotyledon fibres in an amount of more than 6 weight percent of the total weight of the composition on a dry basis,
for the manufacture of a nutritional composition for lowering serum levels of glucose and/or total cholesterol and/or LDL-cholesterol and/or triglycerides in a diabetic subject.

Phytoestrogen compounds are naturally occurring plant substances, which are either structurally or functionally similar to 17β-estradiol or generate estrogenic effects. Phytoestrogens consist of a number of classes including isoflavones, coumestans, lignans and resorcylic acid lactones. Examples of isoflavones according to the present invention are genistein, daidzein, equol, glycitein, biochanin A, formononetin, and O-desmethylangolesin. The phytoestrogen compounds of a composition according to the present invention are preferably isoflavones, more preferably genistein, daidzein, glycitein and/or equol, yet more preferably genistein and/or daidzein and even more preferably genistein. Genistein and daidzein are found almost uniquely in soybeans. A preferred composition according to the present invention may accordingly comprise a single isoflavone, such as genistein, daidzein, glycitein or equol, or it may comprise at least one isoflavone selected from the group consisting of at least genistein, daidzein, glycitein and equol.

Use of a composition according to the present invention may be effective in treating and/or preventing type 2 diabetes and/or the metabolic syndrome and/or reducing and/or eliminating one or more of the risk factors for cardiovascular diseases associated with diabetes and/or the metabolic syndrome. Accordingly, use of a composition according to the present invention may be effective in preventing, alleviating and/or treating conditions such as e.g. increased serum levels of glucose, hypertriglyceridaemia, hypercholesterolaemia, hypertension, and hyperinsulinaemia in diabetic individuals. Use of a composition according to the present invention may also be capable of reducing, preventing and/or eliminating fatty streak formation and/or fibrous plaque development and/or effective in mediating a regression of one or both of said arteriosclerotic conditions.

Use of a composition according to the present invention may be effective in reducing insulin resistance by stimulating cells or receptors located thereon that are normally stimulated by insulin, but less sensitive to the hormone in a subject diagnosed with type 2 diabetes and/or the metabolic syndrome. Use of a composition according to the present invention may also be effective in stimulating cells comprising a beta-2-adrenergic receptor or a receptor belonging to the class of beta-2-adrenergic receptors. The final phase of type 2 diabetes development is characterised by insulin secretory failure (ISF), and in one presently preferred hypothesis, this failure is at least preventable by a composition according to the present invention effective in stimulating insulin secretion.

Both plasma triglyceride and lipoprotein levels are usually increased in individuals treated for type 2 diabetes and/or the metabolic syndrome, and these increased levels, unless reduced by treatment, are likely to lead to coronary heart disease (CHD). Beta-2-adrenergic receptors are present on many different types of cells including fat cells. The beta-2-adrenergic receptor is involved in the regulation of triglyceride synthesis in fat cells and according to one presently preferred hypothesis, binding of soy peptides and/or a phytoestrogen compound such as e.g. a naturally occurring isoflavone to a beta-2-adrenergic receptor present on a fat cell or in an arterial wall is effective in reducing e.g. the synthesis of triglycerides in fat cells and/or the release of triglycerides into the blood stream and/or reducing the influx of triglycerides into the arterial wall. The soy peptides are e.g. obtainable as a partial hydrolysate of soy protein.

Hypertriglyceridaemia in diabetes has been associated with a variety of changes in circulating lipoproteins, and a composition according to the present invention may be capable of preventing, treating, alleviating and/or eliminating cardiovascular risk factors such as e.g. chylomicronaemia, an increased level of VLDL, an increased level of remnants (VLDL and chylomicrons), and LDL and HDL containing increased levels of triglycerides.

Lipoprotein fractions obtained from type 2 diabetic subjects tend to lose their typical sharp LDL peak and instead have a broad diffuse LDL band termed polydisperse LDL. Dissection of polydisperse LDL reveals that diabetics have an increased serum level of intermediate-density-lipoprotein (IDL), an abnormal LDL peak, and an increase in the amount of small dense LDL. While small dense LDL particles have been associated with CHD in the general population, a similar association in diabetes remains to be established. Accordingly, a composition according to the present invention may be effective in promoting a decreased serum level of intermediate density lipoprotein (IDL), a normal, sharp LDL peak, and a decreased amount of small dense LDL.

Accordingly, diabetic dyslipidaemia of type 2 diabetes is generally associated with abnormalities of apolipoprotein and lipoprotein particle distributions and results in increased plasma VLDL and remnant levels, an increase in the apoE concentration in VLDL and remnants, an increase in the amount of small dense LDL, and an altered HDL particle distribution.

According to one presently preferred hypothesis, use of a composition according to the present invention will alleviate abnormalities associated with apolipoprotein and lipoprotein particle distribution and promote a decreased plasma VLDL and remnant level, a decrease in the apoE concentration in VLDL and remnants, a decrease in the amount of small dense LDL, and a HDL particle distribution similar to that of a comparable non-diabetic, healthy individual.

Hypertriglyceridaemia in diabetes is associated with an increase in the clotting activities of thrombogenic factors such as factor VII and factor X and an increase in the level of the inhibitor of tissue plasminogen activator, PAI-1. The increased inhibitor concentration results in a decreased level of plasminogen synthesis and thus a decreased level of plasminogen stimulated clot lysis. These changes in clotting activities no doubt contribute to the observed procoagulant state in diabetes.
Accordingly, the present invention provides use of a composition, which may be effective in normalising the clotting activities of thrombogenic factors such as factor VII and factor X by e.g. decreasing the increased activity thereof observed in a subject diagnosed as having type 2 diabetes or diagnosed as having an impaired glucose tolerance or a decreased insulin sensitivity. Also, use of a composition according to the present invention may be effective in promoting a decrease in the concentration of the inhibitor of tissue plasminogen activator, PAI-1, which in turn leads to an increased plasminogen stimulated clot lysis. Use of a composition according to the present invention may also be effective in reducing an increased platelet aggregatability and/or mediating directly or indirectly a reduction of the increased level of lipoprotein (a) associated with a procoagulant state in a diabetic condition.

Hyperinsulinaemia is also considered a risk factor for coronary heart disease (CHD) in diabetic subjects due to the association of high insulin levels with increased incidence and mortality rates of CHD. Use of a composition according to the present invention may be effective in lowering serum insulin levels in subjects diagnosed with type 2 diabetes. Diabetic patients having increased endogenous insulin levels, i.e. subjects diagnosed with type 2 diabetes, or having increased peripheral circulating insulin levels as a result of intermittent injections of large amounts of exogenous insulin are particularly prone to hyperinsulinaemia.

Hyperinsulinaemia in both normal persons, persons with the metabolic syndrome and those with type 2 diabetes appears to be related to obesity. Insulin levels are very often increased in both the fasted state and after intake of a diet rich in carbohydrates in obese individuals, irrespective of whether they suffer from a diabetic condition or not. Furthermore, hyperinsulinaemia appears to be directly correlated to the degree of obesity. Accordingly, hyperinsulinaemia is one of the many risk factors for CHD associated with obesity, and insulin may modulate many other obesity-related risk factors. Accordingly, use of a composition according to the present invention may be effective in lowering insulin levels in obese subjects with diabetes or the metabolic syndrome.

In obese subjects diagnosed as diabetic, LDL-particle size is independently correlated with factors such as e.g. serum triglyceride and serum insulin levels. Consequently, it is possible that the extent of adiposity and concomitant insulin resistance in hyperinsulinaemic individuals is associated with the occurrence small dense LDL, independently of hypertriglyceridaemia, which is another diabetic condition also putatively associated with small dense LDL formation. Accordingly, both insulin resistance and hyperinsulinaemia appear to play a central role in the pathogenesis of atherosclerosis in diabetes. Use of a composition according to the present invention may be effective in alleviating and/or treating insulin resistance and/or hyperinsulinaemia.

In one embodiment the present invention provides use of a composition effective in reducing and/or eliminating risk factors for coronary heart disease (CHD) in obese subjects suffering from a diabetic condition and/or the metabolic syndrome.
Consequently, use of a composition according to the present invention may be capable of preventing, alleviating, treating and/or eliminating hyperinsulinaemia and/or hyperglycaemia and/or hypertension and/or hypertriglyceridaemia and/or hypercholesterolaemia and/or effective in mediating an increase in the low serum levels of HDL-cholesterol.

It is very possible that type 2 diabetes is also associated with insulin resistance and hyperinsulinaemia independently of an increase in abdominal lipids.
Hyperinsulinaemia in turn is associated with dyslipidaemia, i.e. increased VLDL, decreased and altered HDL and increased small dense LDL, and with hypertension, all of which are risk factors for atheriosclerosis. This array of abnormalities and disorders, or a part of thereof, is generally termed the insulin resistance syndrome, or syndrome X, or metabolic syndrome.

Even though there is no internationally agreed definition for the metabolic syndrome, the term as used herein shall be understood to relate to the occurrence in a subject of at least two of the following: i) impaired glucose tolerance, ii) elevated blood pressure, iii) hypertriglyceridaemia and low HDL-cholesterol, iv) insulin resistance, and v) obesity. The occurrence of a condition characterised by one or more of impaired glucose tolerance, elevated blood pressure, hypertriglyceridaemia and low HDL-cholesterol, insulin resistance, and obesity will depend on variables such as sex, age, body weight, physical condition and the like, and general WHO guidelines will generally be adhered to when evaluating the occurrence of any one of the above-mentioned conditions.

In one embodiment, use of a composition according to the present invention may be capable of effectively decreasing and/or eliminating increased serum levels of VLDL and/or LDL, and/or increasing decreased serum levels of HDL, and of decreasing and/or eliminating serum LDL levels including serum levels of small dense LDL. Use of a composition according to the present invention may also be capable of reducing an elevated level of small, dense LDL-particles and/or reducing an elevated ratio of LDL-apoB to LDL-cholesterol and/or preventing, treating or alleviating hypertension.

Hyperinsulinaemia in itself may well be capable of affecting the arterial wall either directly or indirectly by promoting or facilitating the promotion of changes similar to those leading to severe atherogenesis. Insulin may well promote both arterial smooth muscle cell proliferation and cholesterol ester accumulation in the arterial wall. Use of a composition according to the present invention may in one embodiment be effective in preventing, alleviating, eliminating and/or treating fatty streak formation, fibrous plaque development, complicated lesion formation, thrombosis, platelet aggregation and/or myocardial infarction. Use of a composition according to the present invention may also be capable of suppressing any effect, that would otherwise generate an increased turnover of arterial smooth muscle cells, i.e. an enhanced arterial smooth muscle cell proliferation, and/or lead to an increased cholesterol ester accumulation in the arterial wall.

Since insulin can be expected to be capable, either in combination with other compounds such as additional growth factors, or on its own, of increasing the levels of intracellular cholesterol, by e.g. increasing a delivery of LDL-cholesterol via the LDL-receptor, and concomitantly therewith increase an endogenous biosynthesis of cholesterol that makes yet more cholesterol available for new membrane synthesis in the cell proliferation process, it is an object of the present invention to counteract any increased activity including any insulin stimulated increased activity of the LDL-receptor.

It is also possible that insulin and other growth factors have the potential to promote the accumulation of cholesterol intracellularly. This may in fact well occur in a diabetic subject and more generally under conditions when cells are stimulated, but cannot proliferate normally. Accordingly, use of a composition of the present invention may also be capable of alleviating, eliminating and/or treating any decrease, including any insulin mediated decrease, in the HDL receptor-mediated cholesterol efflux. Accordingly, use of a composition according to the present invention may be capable of reducing and/or eliminating any enhanced retention of intracellular cholesterol caused by a decreasing HDL receptor-mediated cholesterol efflux.

Modifications to lipoproteins are another risk factor for cardiovascular disease in diabetes. The modification characterised by protein glycosylation is associated with diabetes, and glycosylated lipoproteins such as e.g. LDL, IDL, VLDL and HDL can be expected to be functionally abnormal. Accordingly, the accumulation of glycosylated LDL in the plasma of a diabetic subject can be perceived to enhance cholesterol ester accumulation. Also, glycosylation of HDL can be expected to impair the ability of HDL binding to the HDL receptor. This impaired binding is likely to reduce the level of intracellular cholesterol efflux. Accordingly, glycosylated HDL may well be another factor potentially contributing to the accumulation of cholesterol in the arterial cell wall. Use of a composition according to the present invention may be effective in preventing, alleviating, treating, reducing and/or eliminating lipoprotein glycosylation in a diabetic subject. In addition, use of a composition according to the present invention may also be effective in preventing lipoprotein modification caused e.g. by-oxidation, chemical modification such as chemical cross-linking, or modifications caused by an alteration in the lipid composition of the lipoprotein, such as any increase or decrease in the content of triglycerides, cholesterol esters, free cholesterol, and apolipoproteins.

Glycosylated lipoproteins have been suggested to be the subject of further processing leading to the formation of hyperglycosylated compounds. Glycosylation and hyperglycosylation of proteins including lipoproteins in both plasma and the arterial wall can also be expected to be a risk factor for cardiovascular disease including arteriosclerosis in diabetic subjects. Accordingly, use of a composition according to the present invention may be capable of preventing, treating, reducing, alleviating and/or eliminating the accumulation of hyperglycosylated proteins in both serum and cells of the arterial wall. By doing so, the composition is acting to decrease the amount of LDL becoming "trapped" in the arterial wall due to the high degree of glycosylation of arterial wall proteins. Use of a composition according to the present invention may also be effective in alleviating and/or preventing any change to the endothelial cell wall that increase LDL "trapping", and it may be effective in restoring the formation of cells with normal permeability and adhesion parameters.

Lipoprotein glycosylation, hyperglycosylation, oxidation and/or auto-oxidative glycosylation, are risk factors for cardiovascular disease such as arteriosclerosis in diabetes. Accordingly, use of a composition according to the present invention may be effective in eliminating, preventing, alleviating, treating and/or reducing any incidence of lipoprotein glycosylation, hyperglycosylation, oxidation and/or auto-oxidative glycosylation. According to one presently preferred hypothesis, the phytoestrogen compound of a composition according to the present invention is capable of counteracting incidences. The phytoestrogen compound may also be capable of preventing, reducing and/or eliminating the formation of e.g. free radicals that are likely to be involved in such processes, and a composition according to the present invention may be effective in being, promoting, and/or facilitating the formation of an effective antioxidant defence system for counteracting glycosylation, hyperglycosylation, oxidation and/or auto-oxidative glycosylation of serum proteins and proteins including lipoproteins of the arterial cell wall.

Since oxidative stress is a characteristic of diabetes and possibly a contributory factor to among others lipoprotein oxidation and/or glycosylation, and since no efficient antioxidant protection exists due to e.g. significantly decreased levels in diabetic subjects of antioxidants such as e.g. ascorbic acid, use of a composition according to the present invention may be effectively acting as an antioxidant in preventing lipoprotein oxidation and/or glycosylation.

Use of a composition according to the present invention may be effectively acting as an antioxidant in preventing lipoprotein oxidation and/or glycosylation. By the term auto-oxidative glycosylation, or glycoxidation, is understood a reaction catalysed e.g. by reducing sugars that leads to an oxidative modification and/or cross-linking of proteins. The rate of such a process can be expected to be increased in the presence of high glucose concentrations since the oxidising potential is significantly increased under such circumstances. An increased production of free radicals and lipid peroxidation may also contribute to the formation of auto-oxidative glycosylated lipoproteins and this contribution may also be effectively prevented and/or eliminated by use of a composition according to the present invention.

According to another presently preferred hypothesis, the binding of a phytoestrogen compound, such as e.g. isoflavones, optionally in combination with soy peptides preferably provided by hydrolysis of soy protein, to a receptor in the arterial wall, such as e.g. the estrogen receptor, or an estrogen-like receptor, is involved in or effective in controlling uptake of cholesterol and/or triglycerides in the arterial wall, possibly by regulating the permeability of said wall and/or the mechanism of cholesterol and/or triglyceride transport across cellular membranes. Consequently, the binding of isoflavones such as e.g. genistein and/or daidzein to a receptor in the arterial wall may prevent cholesterol and/or triglycerides from entering the arterial wall, or reduce and/or substantially eliminate the amount of cholesterol and/or triglycerides that enters the arterial wall. Receptor binding of isoflavones in the arterial wall is particularly effective in controlling, preventing and/or eliminating fatty streak formation and/or fibrous plaque development and/or effective in mediating a regression of one or both of said arteriosclerotic conditions.

According to a particularly preferred hypothesis, binding of isoflavones such as e.g. genistein and/or daidzein to a receptor in the arterial wall, preferably an estrogen receptor or an estrogen-like receptor, results in an increased nitric oxide synthesis in the endothelial cells of the arterial wall. Nitric oxide is known to exert anti-arteriosclerotic effects including inhibition of platelet adhesion and aggregation, and of smooth muscle cell proliferation. Soy peptides obtainable by hydrolysis of soy protein may participate in the binding of isoflavones to an estrogen receptor or an estrogen-like receptor or the soy peptides may themselves bind to said receptor and exert an action leading to an increased nitric oxide synthesis.

In one presently preferred hypothesis, the establishment of an oxidative potential occurs concomitantly with, and is very likely caused by, a decrease in cellular antioxidant defence systems. This hypothesis is supported by the fact that e.g. ascorbic acid concentrations are decreased in many diabetic individuals. Accordingly, use of a composition according to the present invention may be effective in acting as an antioxidant. This action reduces and/or eliminates LDL, VLDL, IDL and/or HDL susceptibility to oxidation. Concomitantly with a direct anti-oxidative effect, use of a composition according to the present invention may also lower the increased serum glucose levels and by doing so, use of a composition according to the present invention may be effective in reducing the oxidising potential causing and/or contributing to oxidative stress.

Furthermore, use of a composition according to the present invention may also be effective in reducing an enhanced susceptibility to endothelial injury and/or for alleviating and/or restoring and/or improving an inefficient endothelial cell repair mechanism. One effect of such an action exerted by use of a composition according to the present invention is to direct macrophage development away from foam cell formation and to increase the potential of generating arterial smooth muscle cells.

The unique dyslipidaemia associated with type 2 diabetes is a major risk factor for cardiovascular disease, and prevention, alleviation, reduction and/or elimination of dyslipidaemia in diabetic subjects is a prime objective of use of a composition according to the present invention for administration to a diabetic individual. Another important objective of such an administration is the development in a diabetic subject of a gradually reduced insulin resistance and/or a gradually improved glucose tolerance. Since increasing insulin resistance and impaired glucose tolerance are key elements in the progression of type 2 diabetes, the same factors most also be a natural focus of any preventive treatment.

In another presently preferred hypothesis, a composition according to the present invention will promote and/or mediate a reduction in arterial wall thickness and lead to a reduction in the amount of LDL entering the wall. It is believed that an increased thickness of the arterial wall is positively associated with an increased uptake of LDL-particles that are likely to either aggregate or oxidize within the cells of the arterial wall.

Also, use of a composition according to the present invention may be capable of reducing, eliminating and/or preventing the formation of increased serum levels of lipoprotein (a) in a diabetic subject. Lipoprotein (a) levels may primarily be genetically determined, and no current cardiovascular medications are thought effective in lowering serum levels of lipoprotein (a).

### DETAILED DESCRIPTION OF THE INVENTION

A composition for use according to the present invention comprises a novel combination of soy protein, preferably isolated soy protein, a phytoestrogen compound, preferably naturally occurring isoflavones, and dietary fibres, preferably soybean fibres, more preferably soy cotyledon fibres.

The soy protein can be provided by isolated soy protein, soy protein concentrate, soy flour or the like or any combination thereof. Isolated soy protein is preferred.

Isolated soy protein is the major proteinacious fraction of soybeans. It is prepared from high quality, dehulled, defatted soybeans by removing a preponderance of the non-protein components resulting in an isolated soy protein fraction which shall contain at least 90 percent protein (N x 6.25) on a moisture free basis. The preparation takes place through a series of steps in which the soybean protein portion is separated from the rest of the soybean. The removal of carbohydrate results in a product, which is essentially bland in flavour and therefore particularly useful in a nutritional composition for humans.

Soy protein concentrates are made by removing most of the oil and water-soluble non-protein constituents from defatted and dehulled soybeans. In the present context a soy protein concentrate shall preferably contain at least 65 percent protein on a moisture-free basis.

The soy protein can also be provided by soy flour, which can be full-fat or defatted soy flour. Full-fat soy flour comes from whole, dehulled soybeans that have been ground into a fine powder and, as the name implies, still contains the fat naturally found in soybeans. Defatted soy flour comes from whole, dehulled, defatted soybeans that have been ground into a fine powder. Soy flour is approximately 50 percent soy protein on a dry weight basis in the present context.

The soy protein used in a composition for use according to the present invention should preferably supply all the essential amino acids in the amounts required for humans. Preferably, the soy protein should also meet or exceed the essential amino acid requirement pattern for children and adults as established by the Food and Agricultural Organisation, World Health Organisation and United Nations University (FAO/WHO, UNU). Furthermore, the preferred soy protein should be comparable in digestibility to milk, meat, fish, and egg protein. Finally, the preferred soy protein shall be effective in maintaining nitrogen balance when consumed at the recommended protein intake level.

Preferred isolated soy protein products meeting the foregoing requirements are supplied by Protein Technologies International, Inc. under the brand name SUPRO®. SUPRO® isolated soy proteins are supplied in many different qualities and SUPRO® XT 12C is one particularly preferred quality. The currently most preferred quality is termed SUPRO® FXP-HO159.

The soy protein is preferably the main or sole protein source in a nutritional composition according to the present invention. However, parts of the protein source may be provided by other proteins such as e.g. skimmed milk, preferably as a powder, and other vegetable or animal proteins including diary proteins. Preferably, at least 45 weight percent, such as 50 weight percent, for example at least 60 weight percent, such as at least 70 weight percent, for example at least 75 weight percent, such as at least 80 weight percent, for example at least 85 weight percent, such as at least 90 weight percent, for example at least 95 weight percent, such as at least 98 weight percent of the total protein content of the composition is soy protein, and more preferably substantially all of the protein is soy protein.

In a preferred embodiment of the invention the soy protein is provided by isolated soy protein. In this embodiment, preferably at least 50 weight percent, for example at least 60 weight percent, such as at least 70 weight percent, for example at least 75 weight percent, such as at least 80 weight percent, for example at least 85 weight percent, such as at least 90 weight percent, for example at least 95 weight percent, such as at least 98 weight percent of the total protein content of the composition is isolated soy protein, and more preferably substantially all of the protein is provided by isolated soy protein.

The total protein content of a composition for use according to the present invention provides at least 15 percent of the total energy content of the composition, for example 18 percent, such as at least 20 percent, for example at least 22 percent, such as at least 25 percent, for example at least 28 percent, such as at least 30 percent, for example at least 32 percent, such as at least 35 percent, for example at least 38 percent, such as at least 40 percent, for example at least 42 percent, such as at least 45 percent, for example at least 48 percent, such as at least 50 percent of the total energy content of the composition, and preferably less than 90 percent of the total energy content of the composition.

Phytoestrogen compounds according to the present invention are defined as naturally occurring plant substances, said substances being either structurally or functionally similar to 17β-estradiol or generating estrogenic effects. Phytoestrogens consist of a number of classes including isoflavones, coumestans, lignans and resorcylic acid lactones. Examples of isoflavones according to the present invention are genistein, daidzein, equol, glycitein, biochanin A, formononetin, and O-desmethylangolesin. The phytoestrogen compounds of a composition for use according to the present invention are preferably isoflavones, more preferably genistein, daidzein, glycitein and/or equol, yet more preferably genistein and/or daidzein, and even more preferably genistein. A preferred composition according to the present invention may accordingly comprise a single isoflavone, such as genistein, daidzein, glycitein or equol, or it may comprise at least one isoflavone selected from the group consisting of at least genistein, daidzein, glycitein and equol. When present in the plant the isoflavones are mainly in a glucoside form, i.e. attached to a sugar molecule. This glucoside form can be deconjugated to yield a so-called aglycone form, which is the biologically active species. A composition according to the present invention may comprise isoflavones in glucoside and/or aglycone forms regardless of whether the deconjugation to the aglycone form has taken place biologically, *in vitro* or by any other means whereby the isoflavones are included in a composition according to the present invention or if the aglycone forms are the native form of the isoflavones.

The phytoestrogen compound is preferably present in an amount of at least about 0.16 weight percent of the soy protein content, such as at least about 0.18 weight percent, for example at least about 0.20 weight percent, such as at least about 0.22 weight percent, for example at least about 0.24 weight percent, such as at least about 0.25 weight percent, for example more than about 0.25 weight percent, such as at least about 0.26 weight percent, for example at least about 0.28 weight percent, such as at least about 0.30 weight percent, for example at least about 0.32 weight percent, such as at least about 0.33 weight percent, for example more than about 0.33 weight . percent, such as at least about 0.35 weight percent, for example at least about 0.40 weight percent, such as at least about 0.45 weight percent, for example at least about 0.50 weight percent, such as at least about 0.55 weight percent, for example at least about 0.60 weight percent, such as at least about 0.65 weight percent, for example at least about 0.70 weight percent, such as at least about 0.75 weight percent, for example at least about 0.80 weight percent, such as at least about 0.85 weight percent, for example at least about 0.90 weight percent, such as at least about 1.0 weight percent of the soy protein content, and preferably less than 2.50 weight percent of the soy protein content.

In the past, the downstream processing techniques used in the preparation of soy proteins have included steps that removed and/or destroyed isoflavones. Methods are available today, which provide soy protein products with high, fixed levels of naturally occurring isoflavones. The isoflavones for use according to the present invention in glucoside and/or aglycone forms can be included in a composition for use according to the present invention as part of such soy protein products and/or by themselves and/or as part of any other composition comprising isoflavones.

The dietary fibres used in the present invention should preferably comprise a mixture of insoluble fibres and water-soluble fibres also referred to as soluble fibres. Soluble fibres have a lowering effect on blood cholesterol levels. Examples of dietary fibres comprising soluble fibres are fibres from apples, bananas, oranges, carrots, oats, and soybeans. The dietary fibres preferably comprise soluble fibres in an amount of about 5 weight percent, such as about 10 weight percent, for example about 15 weight percent, such as about 20 weight percent, for example about 25 weight percent, such as about 30 weight percent, for example about 35 weight percent, such as about 40 weight percent, for example about 45 weight percent, such as about 50 weight percent, for example about 55 weight percent, such as about 60 weight percent, for example about 65 weight percent, such as about 70 weight percent, for example about 75 weight percent, such as about 80 weight percent, for example about 85 weight percent, such as about 90 weight percent, for example about 95 weight percent. The dietary fibres used in the present invention are preferably soybean fibres, more preferably soy cotyledon fibres. Such fibres are derived from dehulled and defatted soybean cotyledon and are comprised of a mixture of soluble and insoluble fibres. Soy cotyledon fibres are distinctly different from soybean fibres derived from soy hulls as well as other fibre sources. Soy cotyledon fibres are bland tasting, contain no cholesterol, are low in fat and sodium, and they have good water-binding properties and low caloric content.

Soy cotyledon fibres supplied in a fat-modified and low-cholesterol diet are known to further lower serum cholesterol levels in a subject suffering from mild to severe hypercholesterolaemia. The effect is a lowering of the serum levels of total cholesterol including a lowering of LDL-cholesterol. However, HDL-cholesterol and total triglycerides are not significantly affected by soy cotyledon fibres. Soybean fibres, in particular soy cotyledon fibres, are believed to provide a synergistic effect in combination with soy protein and/or with a phytoestrogen compound, such as naturally occurring isoflavones, or to exert a potentiating effect on the soy protein and/or the phytoestrogen compound, said synergistic or potentiating effect being effective in lowering serum lipid and cholesterol levels in subjects having normal as Well as elevated serum levels of total cholesterol and total triglycerides.

Without wishing to be bound by any specific theory it is presently believed that both soluble dietary fibres (working as nutrients) and insoluble dietary fibres (working as bulking agents), in particular from soybean fibres, more particularly soy cotyledon fibres, provide favourable growth conditions for the microflora in the human gut, which makes the microflora more effective in deconjugating isoflavones in the glucoside form to the aglycone form. Isoflavones in the aglycone form are absorbed faster and to a greater extent in the human gut than isoflavones in the glucoside form, and isoflavones in the aglycone form are the biologically more active species. In view hereof it can be understood that administration of a combination of soy proteins, a high, fixed level of isoflavones and a combination of soluble and insoluble fibres is effective in providing an increased uptake of isoflavones.

The amount of dietary fibres of the total weight of a composition according to the present invention on a dry basis is preferably more than 6 weight percent, for example at least 7 weight percent, such as at least 8 weight percent, for example at least 9 weight percent, such as at least 10 weight percent, for example at least 11 weight percent, such as at least 12 weight percent, for example at least 13 weight percent, such as at least 14 weight percent, for example at least 15 weight percent, such as at least 16 weight percent, for example at least 17 weight percent, such as at least 18 weight percent, for example at least 19 weight percent, such as at least 20 weight percent, and preferably less than 50 weight percent.

Preferred amounts of dietary fibres as a weight percent of the content of soy protein, shall be in the range of from about 10 to 100 weight percent, and preferred amounts are in the range of from 15 to 90 weight percent, such as from 20 to 80 weight percent, for example 25 weight percent, such as 30 weight percent, for example 33 weight percent, such as 35 weight percent, for example 40 weight percent, such as 50 weight percent, for example 60 weight percent, such as 70 weight percent, for example 75 weight percent.

. Accordingly, the weight ratio of soy protein to dietary fibres is from about 1.0 to about 10.0, preferably more than about 1.0, for example about 1.25, such as at least about 1.5, for example at least about 1.75, such as at least about 2.0, for example at least about 2.25, such as at least about 2.5, for example at least about 2.75, such as at least about 3.0, for example at least about 3.25, such as at least about 3.5, for example at least about 3.75, such as at least about 4.0, for example at least about 4.25, such as at least about 4.5, for example at least about 4.75, such as at least about 5.0, for example at least about 5.5, such as at least about 6.0, for example at least about 7.5.

The preferred daily dosage of soybean fibres is from at least 1 g to about 100 g soybean fibres, for example from at least 2 to about 75 g soybean fibres, such as from at least 3 g to about 50 g, for example from at least 4 g to about 40 g, such as from at least 5 to about 30 g, such as from at least 10 g to about 20 g soybean fibres.

Preferred soy cotyledon fibre products manufactured by Protein Technologies International, Inc. are marketed under the brand name of FIBRIM®. Among the various soybean fibres produced under the FIBRIM® brand, FIBRIM® 1020 is particularly preferred because of a particularly pleasant mouth feel and dispersability for dry blended beverage applications. FIBRIM® 2000 is presently preferred for use in ready-made liquids.

Two compositions of isolated soy protein and soy cotyledon fibre are preferred in order to maximise the content of soy protein and isoflavones contained therein namely SUPRO® FXP-HO159 and FIBRIM® 1020 for dry blended beverage applications and SUPRO® FXP-HO159 and FIBRIM® 2000 for use in ready made liquids.

A composition for use according to the present invention may optionally comprise a carbohydrate source, a fat source, flavouring agents, vitamins, minerals, electrolytes, trace elements and other conventional additives. The nutritional composition according to the present invention may in one embodiment also comprise one or more flavouring agents such as cocoa, vanilla, lime, strawberry or soup flavours, such as mushroom, tomato or bouillon, and/or and sweeteners such as aspartame as well as other additives such as xanthan gum.

When a carbohydrate source is present in a composition for use according to the present invention, it is preferably present in an amount of less than 30 weight percent such as less than 25 weight percent of the composition. Preferably, the amount of carbohydrate amounts to at least 5 weight percent, more preferred at least 10 weight percent, and most preferred at least 15 weight percent, of the composition. Lecithinated fat reduced cacao is particularly preferred. Other preferred carbohydrates for use in a composition according to the present invention are polydextrose or saccharose, but these should be limited using other sweeteners like e.g. aspartame.

When a fat source is present in a composition for use according to the present invention, it is usually present in an amount from 0.5 to 10 weight percent, preferably 1 to 9 weight percent, such as from 1.5 to 8 weight percent, for example from 2 to 7 weight percent, such as from 2.5 to 6 weight percent of the composition. The fat source will preferably comprise polyunsaturated fatty acids and monounsaturated fatty acids and optionally also saturated fatty acids. Soy lecithins and α-linolenic acids are particularly preferred. The amount of polyunsaturated fatty acids and monounsaturated fatty acids, including the essential fatty acids, may range from 35 to 50, preferably 38 to 44, weight percent of the total amount of the fat source. The essential fatty acids are also called omega-6 and omega-3 fatty acids and include linolic acid and/or linolenic acid (α-linolenic acid). The amount of saturated fatty acids may be from 20 to 30 weight percent, preferably 22 to 26 weight percent, of the total amount of fat.

Vitamins and minerals may optionally be added to a composition for use according to the present invention in accordance with the limits laid down by health authorities. The vitamins will typically include A, B1, B2, B12, folic acid, niacin, panthotenic acid, biotin, C, D, E and K. The minerals will typically include iron, zinc, iodine, copper, manganese, chromium and selenium. Electrolytes, such as sodium, potassium and chlorides, trace elements and other conventional additives may also be added in recommended amounts.

A preferred composition can be obtained by mixing:

| | Content per 100 gram (%) |
|---|---|
| Isolated soy protein (SUPRO® FXP-HO159) | 50.00 |
| Soybean fibres (FIBRIM® 1020) | 16.70 |
| Carbohydrates | 18.20 |
| Lecithinated fat reduced cocoa | 9.30 |
| Soy lecithin | 3.55 |
| Flavourings | 1.25 |
| Xanthan gum | 0.50 |
| Aspartame | 0.50 |

A composition for use according to the present invention may be used as a food for special dietary use, preferably for lowering serum levels of glucose and/or for lowering serum levels of insulin and/or for lowering total serum cholesterol and/or LDL-cholesterol and/or triglyceride levels and/or for increasing glucose tolerance and/or insulin sensitivity and/or for preventing and/or alleviating and/or treating impaired glucose tolerance and/or insulin secretory failure in diabetic subjects and/or for preventing and/or alleviating and/or treating an arteriosclerotic condition by reducing the influx of lipoproteins and/or cholesterol and/or triglycerides into the endocelium of the arterial wall of a diabetic subject suffering from a cardiovascular disease. For example, from one to three daily meals of ordinary food can be supplemented or replaced by a composition according to the present invention. Hereby, significant reductions in serum levels of total cholesterol and LDL-cholesterol and triglyceride can be obtained, as well as an improvement of HDL/LDL-cholesterol ratio and/or an increase in serum HDL-cholesterol levels. The composition may provide from 50 to 250 kcal per serving.

The present invention also provides a composition for use according to the invention in the form of a micronutrient. In this connection a micronutrient is a nutritional supplement and/or a pharmacological composition and/or a medicament comprising i) a synthetic phytoestrogen-like compound capable of binding to an estrogen receptor or an estrogen-like receptor, and/or ii) a naturally occurring, plant-extractable compound in an amount, on a weight per weight basis, in excess of the amount of said compound, when it is present in a natural host such as a plant cell from which the compound can be extracted or isolated, and optionally iii) soy peptides obtainable from a partial hydrolysis of soy protein.

The naturally occurring, plant-extractable compound is preferably but not limited to compounds capable of binding to an estrogen receptor, an estrogen-like receptor, a beta-2-adrenergic receptor or a receptor belonging to the class of beta-2-adrenergic receptors. When the naturally occurring compounds are isolated from plants such as soybeans, they may be selected from the group at least containing phytoestrogens such as soybean phytoestrogens such as soybean isoflavones, soy protein or fragments thereof, e.g. peptides or amino acid sequences, soybean fibres, lecithin, linolenic acid, an antioxidant, a saponin, a lignan, a protease inhibitor, a trypsin inhibitor, and a tyrosine kinase inhibitor. Additional constituents of the micronutrient may preferably be selected among a DNA topoisomerase inhibitor, a ribosome kinase inhibitor, a growth control factor such as e.g. epidermal growth factor, transforming growth factor alpha, platelet derived growth factor, and preferably any growth control factor controllable by a tyrosine kinase activity. The micronutrient may also comprise ormeloxifene and/or levormeloxifene as described by among others Holm et al. (1997) in Arteriosclerosis, Thrombosis, and Vascular Biology 17 (10), 2264 - 2272, and in Clinical Investigation 100 (4), 821 - 828. When the naturally occurring, compound is an isoflavone, the isoflavone may have been deconjugated to the aglycone form either biologically or *in vitro* prior to the incorporation in the micronutrient.

In one particularly preferred embodiment the present invention provides use of a composition or a micronutrient according to the present invention in combination with a functional food ingredient comprising a sterol, preferably an ingredient selected from the group consisting of a stanol ester, a tocotrienol, a mevinolin, and a phytosterol compound such as e.g. campesterol, sitosterol or stigmasterol, or a combination thereof.

According to one preferred embodiment, a composition or a micronutrient for use according to the present invention is for use as a functional food ingredient. A composition or a micronutrient for use according to the present invention may also be administered as a probe or by intravenous administration, or in tablet or capsule form. The present invention also provides use of a composition or a micronutrient according to the present invention, as a medicament and/or in the manufacture of a medicament for treating a subject suffering from type 2 diabetes, the metabolic syndrome or cardiovascular diseases associated therewith.

The micronutrient is particularly useful in the prevention and/or treatment of type 2 diabetes, the metabolic syndrome and cardiovascular diseases associated therewith in a diabetic subject.

In one embodiment the present invention provides use of a composition according to the present invention as a medicament or as a dietary preparation for use in preventing, alleviating, eliminating and/or treating type 2 diabetes and/or a cardiovascular disease associated therewith. The present invention also provides the use of a composition according to the present invention for the manufacture of a medicament for preventing, alleviating and/or treating type 2 diabetes and/or a cardiovascular disease in a diabetic subject.

A composition for use according to the present invention as a medicament and/or the use of a composition according to the present invention for the manufacture of a medicament for treating a subject with diabetes and/or the metabolic syndrome and/or a cardiovascular disease associated therewith may be effective in i) lowering serum glucose levels and/or ii) reducing the influx of cholesterol and/or triglycerides into the arterial wall and/or the amount of oxidized LDL-cholesterol present in the arterial wall and/or iii) lowering total serum cholesterol and/or serum LDL-cholesterol and/or serum triglyceride levels and/or serum homocystein levels and/or increasing the HDL/LDL-cholesterol ratio and/or serum HDL-cholesterol levels and/or iv) increasing glucose tolerance and/or insulin sensitivity and/or v) alleviating impaired glucose tolerance and/or insulin secretory failure and/or vi) preventing, alleviating, eliminating and/or treating cardiovascular diseases, such as e.g. hypertriglyceridaemia, hypercholesterolaemia, arteriosclerosis, atherosclerosis, arteriolosclerosis, angina pectoris, thrombosis, myocardial infarction, hypertension, hyperglycaemia, and hyperinsulinaemia, in a diabetic subject.

A composition for use according to the present invention as a medicament and/or the use of a composition according to the present invention for the manufacture of a medicament may also be effective in treating cardiovascular diseases such as e.g. fatty streak formation and/or fibrous plaque development and/or complicated lesion development. Furthermore, a composition for use according to the present invention as a medicament and/or the use of a composition according to the present invention for the manufacture of a medicament may also be effective in treating a procoagulant state and/or an increased activity of clotting factors, insulin resistance, glycosidation and/or oxidation and/or chemical modification of lipoproteins, as well as impaired glucose tolerance.

The use of compositions of the present invention may be useful in preventing and/or treating by therapy type 2 diabetes and/or the metabolic syndrome in a human or animal body, by administration to said human or animal body of a composition according to the present invention in an amount effective in lowering serum glucose levels and/or reducing the influx of cholesterol and/or triglycerides into the arterial wall and/or reducing the amount of oxidized LDL-cholesterol present in the arterial wall and/or lowering serum cholesterol levels and/or lowering LDL-cholesterol levels and/or lowering serum triglyceride levels and/or serum homocystein levels and/or improving glucose tolerance and/or increasing insulin sensitivity and/or alleviating impaired glucose tolerance and/or improving insulin secretion and/or reducing or eliminating fatty streak formation and/or preventing, reducing or eliminating fibrous plaque formation and/or preventing, reducing or eliminating complicated lesion formation and/or preventing, reducing or eliminating the risk of a subject contracting angina pectoris and/or preventing, reducing or eliminating the risk of a subject contracting a myocardial infarction and/or preventing, treating, prophylactically treating, alleviating and/or eliminating hypertension and/or hyperglycaemia and/or hyperinsulinaemia and/or hypercholesterolaemia and/or hypertriglyceridaemia and/or arteriosclerosis and/or atherosclerosis and/or arteriolosclerosis in a diabetic subject.

The period of treatment is preferably in the range of from 1 to 12 months or more, such as from 2 weeks to 9 months, for example from 3 weeks to 6 months, such as from 4 weeks to 4 months, such as from 6 weeks to 3 months. However, the period of treatment shall not be limited to these periods and may e.g. be longer than 12 months, such as e.g. a lifelong treatment in order to prevent and/or alleviate type 2 diabetes and/or a cardiovascular disease in connection therewith.

In another embodiment the present invention provides the use of a composition according to the present invention in the manufacture of a nutritional preparation for lowering serum glucose levels and/or serum cholesterol levels and/or serum LDL-cholesterol levels and/or serum triglyceride levels and/or serum homocystein levels and/or for increasing the HDL/LDL-cholesterol ratio and/or serum HDL-cholesterol levels in a diabetic subject. The nutritional preparation may take any form, which is suitable for human or animal consumption. In one preferred embodiment, the composition is a powdery mixture, which is suspendable, dispersible or emulsifiable in a liquid for human or animal consumption. The liquid is preferably a water-containing liquid such as e.g. water, coffee, tea or juice. For such a purpose, the composition may be packed in a package intended for covering part of or the total nutritional requirement for a defined period of time, such as a period of e.g. three days or a week. The present invention also provides the nutritional preparation in the form of a dietary supplement.

The nutritional preparation in one embodiment of the present invention is preferably a functional food or drink, i.e. a readily obtainable edible or drinkable substance that is supplemented with a composition for use according to the present invention to provide a medical or pharmaceutical effect. Accordingly, the present invention provides a composition according to the present invention for use as a functional food ingredient. Functional foods and drinks are preferably selected from the group consisting of diary products, such as yoghurt and yoghurt ice cream, juice, such as orange juice or tomato juice, ready made liquids for drinking, a spreadable product such as e.g. a margarine or a vegetable or plant extracted oil, a cereal product, such as a traditional breakfast cereal product, nutritional bars, biscuits, bread, soups, such as tomato soup, a meat product, such as a hamburger, a meat substitute product, and a vegetable product. In a further embodiment, a nutritional preparation according to the present invention may be in the form of a ready made liquid or in a powder form or in the form of a troche, a solid composition such as a nutritional bar, a fruit bar, a cookie, a cake, a bread or a muffin.

In another embodiment, a composition for use according to the present invention is a liquid nutritional preparation in a water-containing liquid, in which the solid ingredients are suspended, dispersed or emulgated in an amount of from 10 to 25 weight percent. When the liquid nutritional preparation is intended for drinking, it will usually comprise a flavouring agent as discussed above. However, the liquid nutritional preparation may also be used for probe administration.

In another embodiment, the present invention relates to the use of a composition according to the present invention as a partial or total diet for an overweight subject or an overweight subject suffering from a diabetic condition. Obesity is believed to be one of the major causes of diabetes including type 2 diabetes. Overweight diabetic subjects often have an increased serum cholesterol level and an increased triglyceride level and are therefore more likely to develop cardiovascular diseases. However, the present invention is not limited to treating obese diabetic subjects with an increased risk of contracting a cardiovascular disease, i.e. obese diabetic subjects likely to have increased serum levels of cholesterol and/or triglycerides. A composition according to the present invention also has substantial serum cholesterol, serum LDL-cholesterol and serum triglyceride lowering effect in diabetic subjects that do not also suffer from overweight.

For the purpose of the present invention, subjects having an initial total serum cholesterol level of 5.7 mmol/l or below are considered to have a normal or hypocholesterolemic level, whereas subjects having a total serum cholesterol level above 5.7 mmol/l are considered to be hypercholesterolemic. Accordingly, by treating normocholesterolemic subjects, it is possible to prevent the development of cardiovascular diseases arising from serum cholesterol levels below a concentration of 5.7 mmol/l in diabetic subjects particularly sensitive to developing e.g. arteriosclerosis, or prevent further development of cardiovascular diseases in diabetic patients with previous cardiovascular events.

### EXAMPLE 1

The study was conducted as a crossover study covering 2 x 6 weeks. In the first six weeks, one half of the patients received a composition according to the invention (Abalon®, Nutri Pharma ASA, Oslo) containing 50% isolated soy protein, a high, fixed intake of isoflavones (3.7 mg/g protein) and 16.7% soy cotyledon fibres while the other half received a placebo product with the same amounts of protein and fibre. In the second six week period, the patients having received Abalon® in the first period now received the placebo product and vice versa. Every two weeks the patients were examined for some of a wide range of factors, which participate in the metabolic syndrome and type 2 diabetes. The following Tables I - V summarizes the results of this examination.

**TABLE I**

| | | Treatment sequence | | | | | | | | |
|---|---|---|---|---|---|---|---|---|---|---|
| | | Active/Placebo | | | | Placebo/Active | | | | |
| Variable | Visit | N | Mean | SD | Wilcoxon | N | Mean | SD | Wilcoxon | Mann-Whitney |
| Total chol. mmol/l | Visit 1 | 12 | 5.80 | 0.93 | | 8 | 5.85 | 0.90 | | ns |
| | Visit 3 | 12 | 5.15 | 0.87 | | 8 | 5.44 | 0.77 | | ns |
| | Diff 113 | 12 | 0.65 | 0.44 | 0.0015 | 8 | 0.41 | 0.79 | ns | ns |
| | Visit 4 | 12 | 5.42 | 0.74 | | 8 | 5.51 | 0.72 | | ns |
| | Visit 6 | 12 | 5.46 | 0.98 | | 8 | 5.04 | 0.65 | | ns |
| | Diff 4/6 | 12 | -0.03 | 0.55 | ns | 8 | 0.47 | 0.31 | 0.0156 | 0.0485 |
| | | | | | | | | | | |
| HDL-chol. mmol/l | Visit 1 | 12 | 1.32 | 0.26 | | 8 | 1.20 | 0.19 | | ns |
| | Visit 3 | 12 | 1.42 | 0.43 | | 8 | 1.30 | 0.13 | | ns |
| | Diff 1/3 | 12 | -0.10 | 0.23 | ns | 8 | -0.10 | 0.22 | ns | ns |
| | Visit 4 | 12 | 1.33 | 0.34 | | 8 | 1.29 | 0.14 | | ns |
| | Visit 6 | 12 | 1.36 | 0.43 | | 8 | 1.31 | 0.16 | | ns |
| | Diff 4/6 | 12 | -0.02 | 0.18 | ns | 8 | -0.03 | 0.15 | ns | ns |
| | | | | | | | | | | |
| LDL-chol. mmol/l | Visit 1 | 11 | 3.75 | 0.92 | | 7 | 3.90 | 0.86 | | ns |
| | Visit 3 | 11 | 3.06 | 0.80 | | 8 | 3.44 | 0.59 | | ns |
| | Diff 113 | 11 | 0.69 | 0.36 | 0.0010 | 7 | 0.46 | 0.73 | ns | ns |
| | Visit 4 | 11 | 3.48 | 0.76 | | 8 | 3.46 | 0.54 | | ns |
| | Visit 6 | 11 | 3.25 | 0.83 | | 8 | 2.94 | 0.53 | | ns |
| | Diff 4/6 | 11 | 0.23 | 0.40 | ns | 8 | 0.53 | 0.24 | 0.0078 | ns |
| | | | | | | | | | | |
| Triglycerides mmol/l | Visit 1 | 12 | 1.68 | 1.24 | | 8 | 1.80 | 1.63 | | ns |
| | Visit 3 | 12 | 1.57 | 1.02 | | 8 | 1.56 | 0.65 | | ns |
| | Diff 1/3 | 12 | 0.12 | 0.44 | ns | 8 | 0.24 | 1.03 | ns | ns |
| | Visit 4 | 12 | 1.63 | 1.45 | | 8 | 1.71 | 1.15 | | ns |
| | Visit 6 | 12 | 1.94 | 1.43 | | 8 | 1.72 | 0.96 | | ns |
| | Diff 4/6 | 12 | -0.31 | 0.44 | 0.0225 | 8 | -0.01 | 0.40 | ns | ns |
| | | | | | | | | | | |
| Apolip. B 100 mg/dl | Visit 1 | 12 | 1.07 | 0.21 | | 8 | 1.06 | 0.27 | | ns |
| | Visit 3 | 12 | 0.82 | 0.20 | | 8 | 0.95 | 0.29 | | ns |
| | Diff 1/3 | 12 | 0.26 | 0.12 | 0.0005 | 8 | 0.11 | 0.25 | ns | ns |
| | Visit 4 | 12 | 0.87 | 0.22 | | 8 | 1.11 | 0.21 | | 0.0265 |
| | Visit 6 | 12 | 1.01 | 0.22 | | 8 | 0.93 | 0.17 | | ns |
| | Diff 4/6 | 12 | -0.14 | 0.23 | ns | 8 | 0.19 | 0.14 | 0.0156 | 0.0022 |
| | | | | | | | | | | |
| Homo cystein µmol/l | Visit 1 | 12 | 11.38 | 4.67 | | 8 | 10.68 | 2.40 | | ns |
| | Visit 3 | 12 | 11.48 | 4.74 | | 8 | 11.80 | 2.48 | | ns |
| | Diff 1/3 | 12 | -0.09 | 1.22 | ns | 8 | -1.13 | 0.84 | 0.0234 | ns |
| | Visit 4 | 12 | 10.52 | 2.80 | | 8 | 10.88 | 2.61 | | ns |
| | Visit 6 | 12 | 13.27 | 5.78 | | 8 | 11.70 | 2.75 | | ns |
| | Diff 4/6 | 12 | -2.74 | 3.17 | 0.0005 | 8 | -0.83 | 0.71 | 0.0313 | 0.0163 |
| In this table, Active/Placebo refers to patients having received Abalon® during the first six weeks (visits 1 - 3) and placebo the last six weeks while Placebo/Active refers to the opposite situation; ns is not significant and Wilcoxon and Mann-Whitney are the used statistical methods. | | | | | | | | | | |

**TABLE II**

| | | Active period | | | | Placebo period | | | | Difference | | | |
|---|---|---|---|---|---|---|---|---|---|---|---|---|---|
| Vari. | Visit | N | Mean | SO | Wilcoxon | N | Mean | SD | Wilcoxon | N | Mean | SD | Wilcoxon |
| Total chol. | Visit 1 | 20 | 5.68 | 0.84 | | 20 | 5.59 | 0.81 | | 20 | 0.09 | 0.66 | ns |
| | Visit 3 | 20 | 5.11 | 0.78 | | 20 | 5.45 | 0.88 | | 20 | -0.34 | 0.50 | 0.0041 |
| | Diff 1/3 | 20 | 0.58 | 0.39 | 0.0001 | 20 | 0.14 | 0.68 | ns | 20 | 0.43 | 0.90 | ns |
| | | | | | | | | | | | | | |
| HDL-chol. | Visit 1 | 20 | 1.31 | 0.22 | | 20 | 1.28 | 0.29 | | 20 | 0.03 | 0.19 | ns |
| | Visit 3 | 20 | 1.38 | 0.35 | | 20 | 1.33 | 0.34 | | 20 | 0.04 | 0.14 | ns |
| | Diff 1/3 | 20 | -0.07 | 0.20 | ns | 20 | -0.05 | 0.20 | ns | 20 | -0.02 | 0.24 | ns |
| | | | | | | | | | | | | | |
| LDL-chol. | Visit 1 | 19 | 3.63 | 0.78 | | 18 | 3.64 | 0.80 | | 18 | -0.00 | 0.66 | ns |
| | Visit 3 | 19 | 3.01 | 0.68 | | 19 | 3.33 | 0.72 | | 19 | -0.32 | 0.40 | 0.0044 |
| | Diff 1/3 | 19 | 0.62 | 0.32 | 0.0001 | 18 | 0.32 | 0.54 | 0.0220 | 18 | 0.32 | 0.67 | ns |
| | | | | | | | | | | | | | |
| TG | Visit 1 | 20 | 1.70 | 1.17 | | 20 | 1.70 | 1.49 | | 20 | -0.01 | 0.58 | ns |
| | Visit 3 | 20 | 1.63 | 0.97 | | 20 | 1.79 | 1.17 | | 20 | -0.16 | 0.57 | ns |
| | Diff 1/3 | 20 | 0.07 | 0.42 | ns | 20 | -0.09 | 0.76 | ns | 20 | 0.16 | 0.78 | ns |
| | | | | | | | | | | | | | |
| Apo B | Visit 1 | 20 | 1.09 | 0.21 | | 20 | 0.95 | 0.26 | | 20 | 0.15 | 0.29 | ns |
| | Visit 3 | 20 | 0.86 | 0.19 | | 20 | 0.98 | 0.25 | | 20 | -0.12 | 0.22 | 0.0249 |
| | Diff 1/3 | 20 | 0.23 | 0.13 | 0.0001 | 20 | -0.04 | 0.26 | ns | 20 | 0.27 | 0.35 | 0.0026 |
| | | | | | | | | | | | | | |
| Homo-cys | Visit 1 | 20 | 11.18 | 3.90 | | 20 | 10.58 | 2.58 | | 20 | 0.59 | 1.90 | ns |
| | Visit 3 | 20 | 11.57 | 3.97 | | 20 | 12.68 | 4.71 | | 20 | -1.11 | 1.58 | 0.0040 |
| | Diff 1/3 | 20 | -0.38 | 1.09 | ns | 20 | -2.10 | 2.60 | 0.0001 | 20 | 1.71 | 2.98 | 0.0057 |
| In this table TG = triglycerides, Apo B = apolipoprotein B, Homo-cys = homocysteine, ns = not significant, Wilcoxon is the used statistical method and all units like in Table I | | | | | | | | | | | | | |

**TABLE III**

| | % change | | |
|---|---|---|---|
| Variable | Active | Placebo | Difference |
| Total cholesterol | -10.1 | -2.5 | 7.6 |
| HDL-cholesterol | 5.3 | 3.9 | 1.4 |
| Triglycerides | -4.1 | 5.3 | 9.4 |
| Apolipo. B 100 | -21.1 | 4.2 | 25.3 |
| Homocystein | 3.4 | 19.8 | 16.4 |

**TABLE IV**

| | | Treatment | | | | | | | | |
|---|---|---|---|---|---|---|---|---|---|---|
| | | Active | | | | Placebo | | | | |
| Variable | Visit | N | Mean | SD | Wilcoxon | N | Mean | SD | Wilcoxon | Mann-Whitney |
| Glucose mmol/l | Visit 1 | 12 | 2736 | 872 | | 8 | 2360 | 772 | | 0.1325 |
| | Visit 4 | 12 | 2903 | 1159 | | 8 | 2478 | 1021 | | 0.3749 |
| | Diff 1/4 | 12 | 167 | 472 | 0.4697 | 8 | 117 | 294 | 0.6406 | 0.9079 |
| | | | | | | | | | | |
| Insulin IU/ml | Visit 1 | 12 | 44436 | 28492 | | 8 | 47125 | 19356 | | 0.5120 |
| | Visit 4 | 12 | 45057 | 29561 | | 8 | 42405 | 19488 | | 0.9692 |
| | Diff 1/4 | 12 | 621 | 7682 | 0.8501 | 8 | -4720 | 6019 | 0.0781 | 0.1325 |
| IU = international units, Wilcooon and Mann-Whitney are the used statistical methods | | | | | | | | | | |

**TABLE V**

| | | Treatment sequence | | | | | | | | |
|---|---|---|---|---|---|---|---|---|---|---|
| | | Active/Placebo | | | | Placebo/Active | | | | |
| Variable | Visit | N | Mean | SD | Wilcoxon | N | Mean | SD | Wilcoxon | Mann-Whitney |
| Glucose mmol/l | 3-1 | 12 | 204 | 379 | 0.1099 | 8 | 189 | 223 | 0.0078 | 0.9692 |
| | 6-4 | 12 | 284 | 441 | 0.0269 | 8 | -16 | 147 | 0.8437 | 0.0409 |
| | | | | | | | | | | |
| Insulin IU/ml | 3-1 | 12 | 26323 | 40064 | 0.0005 | 8 | 27078 | 28502 | 0.0078 | 0.7285 |
| | 6-4 | 12 | 28377 | 30798 | 0.0010 | 8 | 26267 | 22637 | 0.0078 | 0.9079 |
| IU = international units, Wilcoxon and Mann-Whitney are the used statistical methods | | | | | | | | | | |

In this study it is shown that Abalon® in the given dosage and for the given time reduces the amount of total cholesterol, reduces the amount of LDL-cholesterol, reduces the amount of Apolipoprotein B 100, and results in a different level of homocystein than placebo. A significant difference between the Abalon® group and the placebo group with regards to total cholesterol was expected, but the low number of patients and the design of the study have probably blurred the results. The same can be said for the levels of glucose and insulin, where a longer period of study probably would have revealed a larger difference between Abalon® and placebo. Other unpublished studies regarding the cholesterol lowering effect of a composition according to the invention shows, that the effect gets more and more pronounced with time (up to four months).

### EXAMPLE 2

The objective of the present study was to examine if a product comprising isolated soy protein, soybean fibres, and a high, fixed level of isoflavones, is significantly more effective in lowering serum levels of LDL-cholesterol and total cholesterol than placebo. The study was conducted as a randomized, double-blind, placebo controlled trial. Fifty-two patients with a mean baseline cholesterol level of 7.6 mmol/l completed a six-week treatment. Twenty-four consumed a composition according to the invention (Abacor®, Nutri Pharma ASA, Oslo) containing isolated soy protein with high, fixed levels of isoflavones, and soy cotyledon fibres (52 g soy protein, 230 mg soy isoflavones, and 15.5 g soy cotyledon fibres, per day). Twenty-eight consumed a product with the same intakes of protein and fibre, based on casein and cellulose (the placebo). The preparations were given as two daily liquid supplements in addition to the patients regular diets. Both groups were controlled one month after stopping taking the preparations.

The mean reduction of LDL-cholesterol in the Abacor® treated group after six weeks was 13.1%, whereas it was 7.8% (p=0.014) in the placebo treated group. The reduction of total cholesterol was also larger in the active compared to the placebo group (8.4% vs. 5.1 %, p=0.049), without correcting for multiple testing. High-density lipoprotein (HDL) cholesterol showed an increase in both the active and placebo groups (6.2% vs. 5.8%). At the one-month follow-up both groups had returned to pretreatment cholesterol levels.

The results show that intake of a product comprising isolated soy protein with high, fixed levels of isoflavones, and soy cotyledon fibres, significantly reduces serum LDL-cholesterol and total cholesterol, and improves the HDULDL-cholesterol ratio. The positive results were achieved in this group of patients after just six weeks of treatment.

## Claims

1. Use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) dietary fibres in an amount of more than 6 weight percent of the total weight of the nutritional composition on a dry basis
for the manufacture of a nutritional composition for lowering serum levels of glucose and/or total cholesterol and/or LDL-cholesterol and/or triglycerides in a diabetic subject.

2. Use according to claim 1 for lowering serum levels of glucose and/or for lowering serum levels of insulin and/or for lowering total serum cholesterol and/or serum LDL-cholesterol and/or triglyceride levels and/or for increasing glucose tolerance and/or insulin sensitivity and/or for preventing and/or alleviating and/or treating impaired glucose tolerance and/or insulin secretory failure in diabetic subjects and/or for preventing and/or alleviating and/or treating an arteriosclerotic condition by reducing the influx of lipoproteins and/or cholesterol and/or triglycerides into the endocelium.of the arterial wall of a diabetic subject suffering from a cardiovascular disease.

3. Use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) dietary fibres in an amount of more than 6 weight percent of the total weight of the nutritional composition on a dry basis
for the manufacture of a medicament for use in preventing, alleviating, eliminating and/or treating type 2 diabetes in a subject.

4. Use according to claim 3 for improved glucose tolerance and/or an increased insulin sensitivity and/or reduced serum glucose levels and/or an improved insulin secretion.

5. Use according to claim 3 for reducing the influx of cholesterol and/or triglycerides into the arterial wall in a diabetic subject.

6. Use according to claim 3 for preventing, alleviating, eliminating and/or treating a cardiovascular disease in a diabetic subject.

7. Use according to claim 6 wherein said cardiovascular disease is selected from the group consisting of hypertriglyceridaemia, hypercholesterolaemia, hypertension, hyperglycaemia, hyperinsulinaemia, arteriosclerosis, atherosclerosis, arteriolosclerosis, angina pectoris, thrombosis, and myocardial infarction.

8. Use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) dietary fibres in an amount of more than 6 weight percent of the total weight of the nutritional composition on a dry basis
for the manufacture of a medicament for treating a subject suffering from type 2 diabetes and/or the metabolic syndrome

9. Use according to claim 8 wherein the medicament is effective in improving glucose tolerance and/or increasing insulin sensitivity and/or lowering serum glucose levels and/or improving insulin secretion.

10. Use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) dietary fibres in an amount of more than 6 weight percent of the total weight of the nutritional composition on a dry basis
for the manufacture of a medicament for treating a cardiovascular disease in a diabetic subject.

11. Use according to claim 10 wherein the medicament is effective in lowering total serum cholesterol levels and/or serum LDL-cholesterol levels and/or serum triglyceride levels and/or serum homocystein levels and/or effective in increasing the serum HDL/LDL-cholesterol ratio and/or HDL-cholesterol levels in a subject.

12. Use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) dietary fibres in an amount of more than 6 weight percent of the total weight of the nutritional composition on a dry basis
in the manufacture of a nutritional preparation for lowering serum glucose levels and/or total serum cholesterol levels and/or serum LDL-cholesterol levels and/or serum triglyceride levels and/or serum homocystein levels and/or for increasing the HDL/LDL-cholesterol ratio and/or serum HDL-cholesterol levels of a diabetic subject.

13. Use according to claim 13 wherein the nutritional preparation is in the form of a dietary supplement.

14. Use of a composition comprising
(a) a soy protein source, selected from isolated soy protein, soy protein concentrate, or soy flour, said soy protein source providing an amount of soy protein, which is at least 45 weight percent of the total protein content of the composition, said total protein content providing at least 15 percent of the total energy content of the composition,
(b) at least one phytoestrogen compound in an amount of more than 0.16 weight percent of the soy protein content of the composition, and
(c) dietary fibres in an amount of more than 6 weight percent of the total weight of the nutritional composition on a dry basis
for the manufacture of a nutritional preparation as a partial or total diet for an overweight subject suffering from a diabetic condition.

15. Use according to any of claims 1-14, wherein the soy protein source is isolated soy protein and the amount of isolated soy protein is at least 50 weight percent of the total protein content.

16. Use according to claim 15, wherein the amount of isolated soy protein is at least 75 weight percent of the total protein content.

17. Use according to claim 16, wherein the amount of isolated soy protein is at least 90 weight percent of the total protein content.

18. Use according to claim 17, wherein substantially all of the protein is isolated soy protein.

19. Use according to claim 15, wherein the soy protein source is soy protein concentrate or soy flour and the amount of soy protein is at least 50 weight percent of the total protein content.

20. Use according to claim 19, wherein the amount of soy protein is at least 75 weight percent of the total protein content.

21. Use according to claim 20, wherein the amount of soy protein is at least 90 weight percent of the total protein content.

22. Use according to claim 21, wherein substantially all of the protein is soy protein.

23. Use according to any of claims 15 to 22, wherein the dietary fibres are soybean fibres.

24. Use according to claim 23, wherein the soybean fibres are soy cotyledon fibres.

25. Use according to any of claims 15 to 24 wherein the phytoestrogen compound is present in an amount of at least about 0.20 weight percent of the soy protein content of the composition.

26. Use according to claim 25 wherein the phytoestrogen compound is present in an amount of at least about 0.30 weight percent of the soy protein content of the composition.

27. Use according to claim 26 wherein the phytoestrogen compound is present in an amount of at least about 0.33 weight percent of the soy protein content of the composition.

28. Use according to claim 27 wherein the phytoestrogen compound is present in an amount of at least about 0.45 weight percent of the soy protein content of the composition.

29. Use according to claim 28 wherein the phytoestrogen compound is present in an amount of at least about 0.75 weight percent of the soy protein content of the composition.

30. Use according to claim 29 wherein the phytoestrogen compound is present in an amount of at least about 1.0 weight percent of the soy protein content of the composition.

31. Use according to any of claims 15 to 30 wherein the phytoestrogen compound is selected among isoflavones.

32. Use according to claim 31 wherein the isoflavones are selected from the group consisting of genistein, daidzein, glycitein and equol.

33. Use according to claim 32 wherein the isoflavones are genistein and/or daidzein.

34. Use according to claim 33 wherein the isoflavone is genistein.

35. Use according to any of claims 31 to 34 wherein some or all of the isoflavones are present in the aglycone form.

36. Use according to any of claims 15 to 35 wherein the dietary fibres are present in an amount of at least 7 weight percent of the composition.

37. Use according to any of claims 15 to 36 wherein the weight ratio of soy protein to dietary fibres is at least about 1.0.

38. Use according to claim 37 wherein the weight ratio of soy protein to dietary fibres is at least about 1.5.

39. Use according to claim 38 wherein the weight ratio of soy protein to dietary fibres is at least about 2.0.

40. Use according to claim 39 wherein the weight ratio of soy protein to dietary fibres is at least about 2.5.

41. Use according to claim 40 wherein the weight ratio of soy protein to dietary fibres is at least about 3.0.

42. Use according to claim 41 wherein the weight ratio of soy protein to dietary fibres is at least about 4.0.

43. Use according to claim 42 wherein the weight ratio of soy protein to dietary fibres is at least about 5.0.

44. Use according to any of claims 15 to 43 further comprising an additional protein source and/or an additional carbohydrate source and/or an additional fat source:

45. Use according to claim 44 wherein the additional fat source is selected among soy lecithins.

46. Use according to claim 44 wherein the additional fat source is selected among polyunsaturated and monounsaturated fatty acids.

47. Use according to any of claims 15 to 45 in the form of a micronutrient.

48. Use according to claim 47 additionally comprising a DNA topoisomerase inhibitor, a ribosome kinase inhibitor, and/or a growth control factor.

49. Use according to claim 48 wherein the growth control factor is a growth control factor controllable by a tyrosine kinase activity.

50. Use according to any of claims 47 to 49 additionally comprising ormeloxifene and/or levormeloxifene.

51. Use according to any of claims 15 to 50 in combination with a functional food ingredient comprising a sterol.

52. Use according to claim 51 wherein the functional food ingredient comprising a sterol is selected from the group consisting of a stanol ester, a tocotrienol, a mevinolin, and a phytosterol compound, or a combination thereof.

53. Use according to any of claims 15 to 52 for use as a functional food ingredient.

54. Use according to claim 53, where the functional food is selected from the group consisting of diary products, juice, ready made liquids for drinking, a spreadable product, a cereal product, nutritional bars, biscuits, bread, soups, meat products, meat substitute products, and a vegetable product.

## Patentansprüche

1. Verwendung einer Zusammensetzung umfassend
(a) eine Sojaproteinquelle ausgewählt aus isoliertem Sojaprotein, Sojaproteinkonzentrat oder Sojamehl, wobei die Sojaproteinquelle eine Menge an Sojaprotein liefert, die mindestens 45 Gew.% des Gesamtproteingehalts der Zusammensetzung ausmacht, und der Gesamtproteingehalt mindestens 15 % des gesamten Energiegehalts der Zusammensetzung ausmacht,
(b) mindestens eine Phytoöstrogenverbindung in einer Menge von über 0,16 Gew.% des Sojaproteingehalts der Zusammensetzung und
(c) Diätfasern in einer Menge von über 6 Gew.% des Gesamtgewichts der Nahrungszusammensetzung auf Trockenbasis zur Herstellung einer Nahrungszusammensetzung zur Senkung des Serumgehalts an Glucose und/oder Gesamtcholesterin und/oder LDL-Cholesterin und/oder Triglyceriden bei Diabetikern.

2. Verwendung nach Anspruch 1 zur Senkung des Serumgehalts an Glucose und/oder zur Senkung des Serumgehalts an Insulin und/oder zur Senkung des Gesamtcholesteringehalts im Serum und/oder des LDL-Cholesteringehalts im Serum und/oder des Triglyceridgehalts im Serum und/oder zur Erhöhung der Glucosetoleranz und/oder Insulinempfindlichkeit und/oder zur Prävention und/oder Linderung und/oder Behandlung von beeinträchtigter Glucosetoleranz und/oder Insulinsekretionsversagen bei Diabetikern und/oder zur Prävention und/oder Linderung und/oder Behandlung eines Arterioskleroseleidens durch Reduktion des Eindringens von Lipoproteinen und/oder Cholesterin und/oder Triglyceriden in das Endothel der Arterienwand eines an einer Herz-Kreislauf-Erkrankung leidenden Diabetikers.

3. Verwendung einer Zusammensetzung umfassend
(a) eine Sojaproteinquelle ausgewählt aus isoliertem Sojaprotein, Sojaproteinkonzentrat oder Sojamehl, wobei die Sojaproteinquelle eine Menge an Sojaprotein liefert, die mindestens 45 Gew.% des Gesamtproteingehalts der Zusammensetzung ausmacht, und der Gesamtproteingehalt mindestens 15 % des gesamten Energiegehalts der Zusammensetzung ausmacht,
(b) mindestens eine Phytoöstrogenverbindung in einer Menge von über 0,16 Gew.% des Sojaproteingehalts der Zusammensetzung und
(c) Diätfasern in einer Menge von über 6 Gew.% des Gesamtgewichts der Nahrungszusammensetzung auf Trockenbasis zur Herstellung eines Medikaments zur Prävention, Linderung, Elimination und/oder Behandlung von Typ-2-Diabetes bei einer Person.

4. Verwendung nach Anspruch 3 für verbesserte Glucosetoleranz und/oder erhöhte Insulinempfindlichkeit und/oder reduzierte Glucosegehalte im Serum und/oder verbesserte Insulinsekretion.

5. Verwendung nach Anspruch 3 zur Reduktion des Eindringens von Cholesterin und/oder Triglyceriden in die Arterienwand eines Diabetikers.

6. Verwendung nach Anspruch 3 zur Prävention, Linderung, Elimination und/oder Behandlung einer Herz-Kreislauf-Erkrankung bei einem Diabetiker.

7. Verwendung nach Anspruch 6, worin die Herz-Kreislauf-Erkrankung ausgewählt ist aus der Gruppe bestehend aus Hypertriglyceridiämie, Hypercholesterinämie, Hypertonie, Hyperglykämie, Hyperinsulinämie, Arteriosklerose, Atherosklerose, Arteriolosklerose, Angina pectoris, Thrombose und Myokardinfarkt.

8. Verwendung einer Zusammensetzung umfassend
(a) eine Sojaproteinquelle ausgewählt aus isoliertem Sojaprotein, Sojaproteinkonzentrat oder Sojamehl, wobei die Sojaproteinquelle eine Menge an Sojaprotein liefert, die mindestens 45 Gew.% des Gesamtproteingehalts der Zusammensetzung ausmacht, und der Gesamtproteingehalt mindestens 15 % des gesamten Energiegehalts der Zusammensetzung ausmacht,
(b) mindestens eine Phytoöstrogenverbindung in einer Menge von über 0,16 Gew.% des Sojaproteingehalts der Zusammensetzung und
(c) Diätfasern in einer Menge von über 6 Gew.% des Gesamtgewichts der Nahrungszusammensetzung auf Trockenbasis zur Herstellung eines Medikaments zur Behandlung einer an Typ-2-Diabetes und/oder Stoffwechselsyndrom leidenden Person.

9. Verwendung nach Anspruch 8, worin das Medikament wirkungsvoll bei der Verbesserung der Glucosetoleranz und/oder Erhöhung der Insulinempfindlichkeit und/oder Senkung des Glucosegehalts im Serum und/oder Verbesserung der Insulinsekretion ist.

10. Verwendung einer Zusammensetzung umfassend
(a) eine Sojaproteinquelle ausgewählt aus isoliertem Sojaprotein, Sojaproteinkonzentrat oder Sojamehl, wobei die Sojaproteinquelle eine Menge an Sojaprotein liefert, die mindestens 45 Gew.% des Gesamtproteingehalts der Zusammensetzung ausmacht, und der Gesamtproteingehalt mindestens 15 % des gesamten Energiegehalts der Zusammensetzung ausmacht,
(b) mindestens eine Phytoöstrogenverbindung in einer Menge von über 0,16 Gew.% des Sojaproteingehalts der Zusammensetzung und
(c) Diätfasern in einer Menge von über 6 Gew.% des Gesamtgewichts der Nahrungszusammensetzung auf Trockenbasis zur Herstellung eines Medikaments zur Behandlung einer Herz-Kreislauf-Erkrankung bei einem Diabetiker.

11. Verwendung nach Anspruch 10, worin das Medikament wirkungsvoll bei der Senkung des Gesamtcholesteringehalts im Serum und/oder des LDL-Cholesterinsgehalts im Serum und/oder des Triglyceridgehalts im Serum und/oder des Homocysteingehalts im Serum und/oder wirkungsvoll bei der Erhöhung des Verhältnisses von HDL/LDL-Cholesterin im Serum und/oder des HDL-Cholesteringehalts im Serum einer Person ist.

12. Verwendung einer Zusammensetzung umfassend
(a) eine Sojaproteinquelle ausgewählt aus isoliertem Sojaprotein, Sojaproteinkonzentrat oder Sojamehl, wobei die Sojaproteinquelle eine Menge an Sojaprotein liefert, die mindestens 45 Gew.% des Gesamtproteingehalts der Zusammensetzung ausmacht, und der Gesamtproteingehalt mindestens 15 % des gesamten Energiegehalts der Zusammensetzung ausmacht,
(b) mindestens eine Phytoöstrogenverbindung in einer Menge von über 0,16 Gew.% des Sojaproteingehalts der Zusammensetzung und
(c) Diätfasern in einer Menge von über 6 Gew.% des Gesamtgewichts der Nahrungszusammensetzung auf Trockenbasis bei der Herstellung einer Nahrungspräparation zur Senkung des Glucosegehalts im Serum und/oder des Gesamtcholesteringehalts im Serum und/oder des LDL-Cholesteringehalts im Serum und/oder des Triglyceridgehalts im Serum und/oder des Homocysteingehalts im Serum und/oder zur Erhöhung des Verhältnisses von HDL/LDL-Cholesterin im Serum und/oder des HDL-Cholesterin-Gehalts im Serum eines Diabetikers.

13. Verwendung nach Anspruch 12, worin die Nahrungspräparation in Form eines diätetischen Zusatzes vorliegt.

14. Verwendung einer Zusammensetzung umfassend
(a) eine Sojaproteinquelle ausgewählt aus isoliertem Sojaprotein, Sojaproteinkonzentrat oder Sojamehl, wobei die Sojaproteinquelle eine Menge an Sojaprotein liefert, die mindestens 45 Gew.% des Gesamtproteingehalts der Zusammensetzung ausmacht, und der Gesamtproteingehalt mindestens 15 % des gesamten Energiegehalts der Zusammensetzung ausmacht,
(b) mindestens eine Phytoöstrogenverbindung in einer Menge von über 0,16 Gew.% des Sojaproteingehalts der Zusammensetzung und
(c) Diätfasern in einer Menge von über 6 Gew.% des Gesamtgewichts der Nahrungszusammensetzung auf Trockenbasis zur Herstellung einer Nahrungspräparation als Teil- oder Gesamtdiät für eine übergewichtige Person, die an Diabetes leidet.

15. Verwendung nach einem der Ansprüche 1 bis 14, worin die Sojaproteinquelle isoliertes Sojaprotein ist und die Menge an isoliertem Sojaprotein mindestens 50 Gew.% des Gesamtproteingehalts ausmacht.

16. Verwendung nach Anspruch 15, worin die Menge an isoliertem Sojaprotein mindesten 75 Gew.% des Gesamtproteingehalts ausmacht.

17. Zusammensetzung nach Anspruch 16, worin die Menge an isoliertem Sojaprotein mindesten 90 Gew.% des Gesamtproteingehalts ausmacht.

18. Verwendung nach Anspruch 17, worin im Wesentlichen das gesamte Protein isoliertes Sojaprotein ist.

19. Verwendung nach Anspruch 15, worin die Sojaproteinquelle Sojaproteinkonzentrat oder Sojamehl ist und die Menge an Sojaprotein mindestens 50 Gew.% des Gesamtproteingehalts ausmacht.

20. Verwendung nach Anspruch 19, worin die Menge an Sojaprotein mindestens 75 Gew.% des Gesamtproteingehalts ausmacht.

21. Verwendung nach Anspruch 20, worin die Menge an Sojaprotein mindestens 90 Gew.% des Gesamtproteingehalts ausmacht.

22. Verwendung nach Anspruch 21, worin im Wesentlichen das gesamte Protein Sojaprotein ist.

23. Verwendung nach einem der Ansprüche 15 bis 22, worin die Diätfasern Sojabohnenfasern sind.

24. Verwendung nach Anspruch 23, worin die Sojabohnenfasern Fasern aus Sojakeimblättern sind.

25. Verwendung nach einem der Ansprüche 15 bis 24, worin die Phytoöstrogen-Verbindung in einer Menge von mindestens etwa 0,20 Gew.% des Sojaproteingehalts der Zusammensetzung anwesend ist.

26. Verwendung nach Anspruch 25, worin die Phytoöstrogen-Verbindung in einer Menge von mindestens etwa 0,30 Gew.% des Sojaproteingehalts der Zusammensetzung anwesend ist.

27. Verwendung nach Anspruch 26, worin die Phytoöstrogen-Verbindung in einer Menge von mindestens etwa 0,33 Gew.% des Sojaproteingehalts der Zusammensetzung anwesend ist.

28. Verwendung nach Anspruch 27, worin die Phytoöstrogen-Verbindung in einer Menge von mindestens etwa 0,45 Gew.% des Sojaproteingehalts der Zusammensetzung anwesend ist.

29. Verwendung nach Anspruch 28, worin die Phytoöstrogen-Verbindung in einer Menge von mindestens etwa 0,75 Gew.% des Sojaproteingehalts der Zusammensetzung anwesend ist.

30. Verwendung nach Anspruch 29, worin die Phytoöstrogen-Verbindung in einer Menge von mindestens etwa 1,0 Gew.% des Sojaproteingehalts der Zusammensetzung anwesend ist.

31. Verwendung nach einem der Ansprüche 15 bis 30, worin die Phytoöstrogen-Verbindung aus Isoflavonen ausgewählt ist.

32. Verwendung nach Anspruch 31, worin die Isoflavone ausgewählt sind aus der Gruppe bestehend aus Genistein, Daidzein, Glycitein und Equol.

33. Verwendung nach Anspruch 32, worin die Isoflavone Genistein und/oder Daidzein sind.

34. Verwendung nach Anspruch 33, worin das Isoflavon Genistein ist.

35. Verwendung nach einem der Ansprüche 31 bis 34, worin ein Teil oder die Gesamtheit der Isolflavone in Aglykonform anwesend sind.

36. Verwendung nach einem der Ansprüche 15 bis 35, worin die Diätfasern in einer Menge von mindestens 7 Gew.% der Zusammensetzung anwesend sind.

37. Verwendung nach einem der Ansprüche 15 bis 36, worin das Gewichtsverhältnis von Sojaprotein zu Diätfasern mindestens etwa 1,0 beträgt.

38. Verwendung nach Anspruch 37, worin das Gewichtsverhältnis von Sojaprotein zu Diätfasern mindestens etwa 1,5 beträgt.

39. Verwendung nach Anspruch 38, worin das Gewichtsverhältnis von Sojaprotein zu Diätfasern mindestens etwa 2,0 beträgt.

40. Verwendung nach Anspruch 39, worin das Gewichtsverhältnis von Sojaprotein zu Diätfasern mindestens etwa 2,5 beträgt.

41. Verwendung nach Anspruch 40, worin das Gewichtsverhältnis von Sojaprotein zu Diätfasern mindestens etwa 3,0 beträgt.

42. Verwendung nach Anspruch 41, worin das Gewichtsverhältnis von Sojaprotein zu Diätfasern mindestens etwa 4,0 beträgt.

43. Verwendung nach Anspruch 42, worin das Gewichtsverhältnis von Sojaprotein zu Diätfasern mindestens etwa 5,0 beträgt.

44. Verwendung nach einem der Ansprüche 15 bis 43, welche weiters eine zusätzliche Proteinquelle und/oder eine zusätzliche Kohlehydratquelle und/oder eine zusätzliche Fettquelle umfasst.

45. Verwendung nach Anspruch 44, worin die zusätzliche Fettquelle ausgewählt ist aus Sojalecithinen.

46. Verwendung nach Anspruch 44, worin die zusätzliche Fettquelle ausgewählt ist aus polyungesättigten und monoungesättigten Fettsäuren.

47. Verwendung nach einem der Ansprüche 15 bis 45 in Form eines Mikronährstoffs.

48. Verwendung nach Anspruch 47, welche zusätzlich einen DNA-Topoisomerase-Hemmer, einen Ribosomenkinase-Hemmer und/oder einen Wachstumskontrollfaktor umfasst.

49. Verwendung nach Anspruch 48, worin der Wachstumskontrollfaktor ein durch Tyrosinkinase-Aktivität steuerbarer Wachstumskontrollfaktor ist.

50. Verwendung nach einem der Ansprüche 47 bis 49, welche zusätzlich Ormeloxifen und/oder Levormeloxifen umfasst.

51. Verwendung nach einem der Ansprüche 15 bis 50 in Kombination mit einem ein Sterin enthaltenden funktionellen Nahrungsinhaltsstoff.

52. Verwendung nach Anspruch 51, worin der ein Sterin enthaltende funktionelle Nahrungsinhaltsstoff ausgewählt ist aus der Gruppe bestehend aus einem Stanolester, einem Tocotrienol, einem Mevinolin und einer Phytosterinverbindung oder einer Kombination davon.

53. Verwendung nach einem der Ansprüche 15 bis 52 zur Verwendung als funktioneller Nahrungsinhaltsstoff.

54. Verwendung nach Anspruch 53, worin das funktionelle Nahrungsmittel ausgewählt ist aus der Gruppe bestehend aus Milchprodukten, Säften, trinkfertigen Flüssigkeiten, streichfähigen Produkten, Getreideprodukten, Nahrungsriegeln, Keksen, Broten, Suppen, Fleischprodukten, Fleischersatzprodukten und Gemüseprodukten.

## Revendications

1. Utilisation d'une composition comprenant :
a) une source de protéine de soja, choisie parmi une protéine de soja isolée, un concentré de protéine de soja ou de la farine de soja, ladite source de protéine de soja fournissant une quantité de protéine de soja qui est d'au moins 45% en poids de la teneur totale en protéine de la composition, ladite teneur totale en protéine fournissant au moins 15% en poids de la teneur énergétique totale de la composition,
b) au moins un composé phyto-oestrogène en quantité de plus de 0,16% en poids de la teneur en protéine de soja de la composition, et
c) des fibres alimentaires en quantité de plus de 6% en poids du poids total de la composition nutritionnelle sur base sèche,
pour la fabrication d'une composition nutritionnelle permettant d'abaisser les niveaux de glucose et/ou de cholestérol total et/ou de cholestérol LDL et/ou de triglycérides dans le sérum chez un sujet diabétique.

2. Utilisation selon la revendication 1 pour abaisser les niveaux de glucose dans le sérum et/ou abaisser les niveaux d'insuline dans le sérum et/ou abaisser les niveaux de cholestérol total dans le sérum et/ou les niveaux de cholestérol LDL et de triglycérides dans le sérum et/ou pour augmenter la tolérance au glucose et/ou la sensibilité à l'insuline et/ou pour empêcher et/ou atténuer et/ou traiter la tolérance à l'insuline dégradée et/ou le manque de sécrétion d'insuline chez les sujets diabétiques et/ou pour empêcher et/ou atténuer et/ou traiter un état athéroscléreux en réduisant l'afflux de lipoprotéines et/ou de cholestérol et/ou de triglycérides dans l'endocellium de la paroi artérielle d'un sujet diabétique souffrant d'une affection cardiovasculaire.

3. Utilisation d'une composition comprenant :
a) une source de protéine de soja, choisie parmi une protéine de soja isolée, un concentré de protéine de soja ou de la farine de soja, ladite source de protéine de soja fournissant une quantité de protéine de soja qui est d'au moins 45% en poids de la teneur totale en protéine de la composition, ladite teneur totale en protéine fournissant au moins 15% en poids de la teneur énergétique totale de la composition,
b) au moins un composé phyto-oestrogène en quantité de plus de 0,16% en poids de la teneur en protéine de soja de la composition, et
c) des fibres alimentaires en quantité de plus de 6% en poids du poids total de la composition nutritionnelle sur base sèche,
pour la fabrication d'un médicament permettant d'empêcher, d'atténuer, d'éliminer et/ou de traiter le diabète de type 2 chez un sujet.

4. Utilisation selon la revendication 3 pour obtenir une meilleure tolérance au glucose et/ou une plus grande sensibilité à l'insuline et/ou une réduction des niveaux de glucose dans le sérum et/ou une meilleure sécrétion d'insuline.

5. Utilisation selon la revendication 3 pour réduire l'afflux de cholestérol et/ou de triglycérides dans la paroi artérielle chez un sujet diabétique.

6. Utilisation selon la revendication 3 pour empêcher, atténuer, éliminer et/ou traiter une affection cardiovasculaire chez un sujet diabétique.

7. Utilisation selon la revendication 6, dans laquelle ladite affection cardiovasculaire est choisie dans le groupe constitué de l'hypertriglycéridémie, de l'hypercholestérolémie, de l'hypertension, de l'hyperglycémie, de l'hyperinsulinémie, de l'artériosclérose, de l'athérosclérose, de l'artériolosclérose, de l'angine de poitrine, de la thrombose et de l'infarctus du myocarde.

8. Utilisation d'une composition comprenant :
a) une source de protéine de soja, choisie parmi une protéine de soja isolée, un concentré de protéine de soja ou de la farine de soja, ladite source de protéine de soja fournissant une quantité de protéine de soja qui est d'au moins 45% en poids de la teneur totale en protéine de la composition, ladite teneur totale en protéine fournissant au moins 15% en poids de la teneur énergétique totale de la composition,
b) au moins un composé phyto-oestrogène en quantité de plus de 0,16% en poids de la teneur en protéine de soja de la composition, et
c) des fibres alimentaires en quantité de plus de 6% en poids du poids total de la composition nutritionnelle sur base sèche,
pour la fabrication d'un médicament permettant de traiter un sujet souffrant d'un diabète de type 2 et/ou d'un syndrome métabolique.

9. Utilisation selon la revendication 8, dans laquelle le médicament est efficace pour améliorer la tolérance au glucose et/ou augmenter la sensibilité à l'insuline et/ou réduire les niveaux de glucose dans le sérum et/ou améliorer la sécrétion d'insuline.

10. Utilisation d'une composition comprenant :
a) une source de protéine de soja, choisie parmi une protéine de soja isolée, un concentré de protéine de soja ou de la farine de soja, ladite source de protéine de soja fournissant une quantité de protéine de soja qui est d'au moins 45% en poids de la teneur totale en protéine de la composition, ladite teneur totale en protéine fournissant au moins 15% en poids de la teneur énergétique totale de la composition,
b) au moins un composé phyto-oestrogène en quantité de plus de 0,16% en poids de la teneur en protéine de soja de la composition, et
c) des fibres alimentaires en quantité de plus de 6% en poids du poids total de la composition nutritionnelle sur base sèche,
pour la fabrication d'un médicament permettant de traiter une affection cardiovasculaire chez un sujet diabétique.

11. Utilisation selon la revendication 10, dans laquelle le médicament est efficace pour abaisser les niveaux de cholestérol total dans le sérum et/ou les niveaux de cholestérol LDL dans le sérum et/ou les niveaux de triglycérides dans le sérum et/ou les niveaux d'homocystéine dans le sérum et/ou efficace pour augmenter le rapport du cholestérol HDL au cholestérol LDL et/ou les niveaux de cholestérol HDL chez un sujet.

12. Utilisation d'une composition comprenant :
a) une source de protéine de soja, choisie parmi une protéine de soja isolée, un concentré de protéine de soja ou de la farine de soja, ladite source de protéine de soja fournissant une quantité de protéine de soja qui est d'au moins 45% en poids de la teneur totale en protéine de la composition, ladite teneur totale en protéine fournissant au moins 15% en poids de la teneur énergétique totale de la composition,
b) au moins un composé phyto-oestrogène en quantité de plus de 0,16% en poids de la teneur en protéine de soja de la composition, et
c) des fibres alimentaires en quantité de plus de 6% en poids du poids total de la composition nutritionnelle sur base sèche,
pour la fabrication d'une préparation nutritionnelle pour abaisser les niveaux de glucose dans le sérum et/ou les niveaux de cholestérol total dans le sérum et/ou les niveaux de cholestérol LDL dans le sérum et/ou les niveaux de triglycérides dans le sérum et/ou les niveaux d'homocystéine dans le sérum et/ou pour augmenter le rapport du cholestérol HDL au cholestérol LDL et/ou les niveaux de cholestérol HDL d'un sujet diabétique.

13. Utilisation selon la revendication 12, dans laquelle la préparation nutritionnelle se présente sous la forme d'un supplément alimentaire.

14. Utilisation d'une composition comprenant :
a) une source de protéine de soja, choisie parmi une protéine de soja isolée, un concentré de protéine de soja ou de la farine de soja, ladite source de protéine de soja fournissant une quantité de protéine de soja qui est d'au moins 45% en poids de la teneur totale en protéine de la composition, ladite teneur totale en protéine fournissant au moins 15% en poids de la teneur énergétique totale de la composition,
b) au moins un composé phyto-oestrogène en quantité de plus de 0,16% en poids de la teneur en protéine de soja de la composition, et
c) des fibres alimentaires en quantité de plus de 6% en poids du poids total de la composition nutritionnelle sur base sèche,
pour la fabrication d'une préparation nutritionnelle en tant que régime partiel ou régime total pour un sujet ayant une surcharge pondérale souffrant d'un état diabétique.

15. Utilisation selon l'une quelconque des revendications 1 à 14, dans laquelle la source de protéine de soja est une protéine de soja isolée et la quantité de protéine de soja isolée est d'au moins 50% en poids de la teneur totale en protéine.

16. Utilisation selon la revendication 15, dans laquelle la quantité de protéine de soja isolée est d'au moins 75% en poids de la teneur totale en protéine.

17. Utilisation selon la revendication 16, dans laquelle la quantité de protéine de soja isolée est d'au moins 90% en poids de la teneur totale en protéine.

18. Utilisation selon la revendication 17, dans laquelle sensiblement toute la protéine est une protéine de soja isolée.

19. Utilisation selon la revendication 15, dans laquelle la source de protéine de soja est un concentré de protéine de soja ou de la farine de soja et la quantité de protéine de soja est d'au moins 50% en poids de la teneur totale en protéine.

20. Utilisation selon la revendication 19, dans laquelle la quantité de protéine de soja est d'au moins 75% en poids de la teneur totale en protéine.

21. Utilisation selon la revendication 20, dans laquelle la quantité de protéine de soja est d'au moins 90% en poids de la teneur totale en protéine.

22. Utilisation selon la revendication 21, dans laquelle sensiblement toute la protéine est une protéine de soja.

23. Utilisation selon l'une quelconque des revendications 15 à 22, dans laquelle les fibres alimentaires sont des fibres de haricots de soja.

24. Utilisation selon la revendication 23, dans laquelle les fibres de haricots de soja sont des fibres de cotylédons de soja.

25. Utilisation selon l'une quelconque des revendications 15 à 24, dans laquelle le composé phyto-oestrogène est présent en quantité d'au moins environ 0,20% en poids de la teneur en protéine de soja de la composition.

26. Utilisation selon la revendication 25, dans laquelle le composé phyto-oestrogène est présent en quantité d'au moins environ 0,30% en poids de la teneur en protéine de soja de la composition.

27. Utilisation selon la revendication 26, dans laquelle le composé phyto-oestrogène est présent en quantité d'au moins environ 0,33% en poids de la teneur en protéine de soja de la composition.

28. Utilisation selon la revendication 27, dans laquelle le composé phyto-oestrogène est présent en quantité d'au moins environ 0,45% en poids de la teneur en protéine de soja de la composition.

29. Utilisation selon la revendication 28, dans laquelle le composé phyto-oestrogène est présent en quantité d'au moins environ 0,75% en poids de la teneur en protéine de soja de la composition.

30. Utilisation selon la revendication 29, dans laquelle le composé phyto-oestrogène est présent en quantité d'au moins environ 1,0% en poids de la teneur en protéine de soja de la composition.

31. Utilisation selon l'une quelconque des revendications 15 à 30, dans laquelle le composé phyto-oestrogène est choisi parmi les isoflavones.

32. Utilisation selon la revendication 31, dans laquelle les isoflavones sont choisies dans le groupe constitué de la génistéine, de la daidzéine, de la glycitéine et de l'équol.

33. Utilisation selon la revendication 32, dans laquelle les isoflavones sont la génistéine et/ou la daidzéine.

34. Utilisation selon la revendication 33, dans laquelle l'isoflavone est la génistéine.

35. Utilisation selon l'une quelconque des revendications 31 à 34, dans laquelle certaines ou toutes les isoflavones sont présentes sous la forme aglycone.

36. Utilisation selon l'une quelconque des revendications 15 à 35, dans laquelle les fibres alimentaires sont présentes en quantité d'au moins 7% en poids de la composition.

37. Utilisation selon l'une quelconque des revendications 15 à 36, dans laquelle le rapport pondéral de la protéine de soja aux fibres alimentaires est d'au moins environ 1,0.

38. Utilisation selon la revendication 37, dans laquelle le rapport pondéral de la protéine de soja aux fibres alimentaires est d'au moins environ 1,5.

39. Utilisation selon la revendication 38, dans laquelle le rapport pondéral de la protéine de soja aux fibres alimentaires est d'au moins environ 2,0.

40. Utilisation selon la revendication 39, dans laquelle le rapport pondéral de la protéine de soja aux fibres alimentaires est d'au moins environ 2,5.

41. Utilisation selon la revendication 40, dans laquelle le rapport pondéral de la protéine de soja aux fibres alimentaires est d'au moins environ 3,0.

42. Utilisation selon la revendication 41, dans laquelle le rapport pondéral de la protéine de soja aux fibres alimentaires est d'au moins environ 4,0.

43. Utilisation selon la revendication 42, dans laquelle le rapport pondéral de la protéine de soja aux fibres alimentaires est d'au moins environ 5,0.

44. Utilisation selon l'une quelconque des revendications 15 à 43, comprenant en outre une source de protéine supplémentaire et/ou une source d'hydrate de carbone supplémentaire et/ou une source de graisse supplémentaire.

45. Utilisation selon la revendication 44, dans laquelle la source de graisse supplémentaire est choisie parmi les lécithines de soja.

46. Utilisation selon la revendication 44, dans laquelle la source de graisse supplémentaire est choisie parmi les acides gras polyinsaturés et monoinsaturés.

47. Utilisation selon l'une quelconque des revendications 15 à 45 sous la forme d'un micronutriment.

48. Utilisation selon la revendication 47, comprenant en outre un inhibiteur de topoisomérase d'ADN, un inhibiteur de kinase de ribosomes et/ou un facteur de contrôle de croissance.

49. Utilisation selon la revendication 48, dans laquelle le facteur de contrôle de croissance est un facteur de contrôle de croissance ajustable par une activité de kinase tyrosine.

50. Utilisation selon l'une quelconque des revendications 47 à 49, comprenant en outre de l'orméloxifène et/ou de la lévorméloxifène.

51. Utilisation selon l'une quelconque des revendications 15 à 50 en combinaison avec un ingrédient alimentaire fonctionnel comprenant un stérol.

52. Utilisation selon la revendication 51, dans laquelle l'ingrédient alimentaire fonctionnel comprenant un stérol est choisi dans le groupe constitué d'un ester de stanol, d'un tocotriénol, d'une mévinoline et d'un composé de phytostérol, ou d'une de leurs combinaisons.

53. Utilisation selon l'une quelconque des revendications 15 à 52 pour un usage comme ingrédient alimentaire fonctionnel.

54. Utilisation selon la revendication 53, dans laquelle l'aliment fonctionnel est choisi dans le groupe constitué des produits lactés, de jus de fruits, de liquides de boisson prêts à consommer, d'un produit à tartiner, d'un produit à base de céréale, de barres alimentaires, de biscuits, de pain, de soupes, de produits carnés, de produits de substitution à la viande et d'un produit végétal.
